(19) **Europäisches Patentamt / European Patent Office / Office européen des brevets**

(11) **EP 3 373 956 B1**

(12) **EUROPEAN PATENT SPECIFICATION**

(45) Date of publication and mention of the grant of the patent:
**04.01.2023 Bulletin 2023/01**

(21) Application number: **16863236.2**

(22) Date of filing: **11.11.2016**

(51) International Patent Classification (IPC):
**A61K 38/43** (2006.01)　　**A61K 38/48** (2006.01)
**A61P 35/00** (2006.01)

(52) Cooperative Patent Classification (CPC):
**C12Y 304/21004; A61K 38/4826; A61P 35/00; C12Y 304/21001**

(86) International application number:
**PCT/AU2016/051082**

(87) International publication number:
**WO 2017/079802 (18.05.2017 Gazette 2017/20)**

(54) **PROENZYME COMPOSITION**

PROENZYMZUSAMMENSETZUNG

COMPOSITION DE PROENZYME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.11.2015　AU 2015904678**
**12.04.2016　US 201662321377 P**

(43) Date of publication of application:
**19.09.2018 Bulletin 2018/38**

(73) Proprietor: **Propanc Pty Ltd**
**Camberwell, Victoria 3124 (AU)**

(72) Inventors:
- **KENYON, Julian**
  **Camberwell, Victoria 3124 (AU)**
- **BRANDT, Ralf**
  **Camberwell, Victoria 3124 (AU)**

(74) Representative: **Weickmann & Weickmann PartmbB**
**Postfach 860 820**
**81635 München (DE)**

(56) References cited:
WO-A1-2011/047434　　WO-A1-2015/070828
WO-A1-2017/177270　　RU-C1- 2 149 021
US-A- 4 514 388　　US-A- 5 858 357

- **PERAN MACARENA ET AL: "A formulation of pancreatic proenzymes provides potent antitumour efficacy: a pilot study focused on pancreatic and ovarian cancer", SCIENTIFIC REPORTS, vol. 7, 25 October 2017 (2017-10-25), XP002790107, ISSN: 2045-2322**
- **NOVAK, J.F. ET AL.: 'Proenzyme Therapy of Cancer' ANTICANCER RESEARCH. vol. 25, 2005, pages 1157 - 1177, XP008156400**
- **KAISEROVA, P. ET AL.: 'Proenzyme therapy of sarcoma S-180 and melanoma B16-F10' JOURNAL OF APPLIED BIOMEDICINE. vol. 12, no. 1, 2014, pages 39 - 47, XP055380384**
- **PERAN, M. ET AL.: 'In vitro treatment of carcinoma cell lines with pancreatic (pro)enzymes suppresses the EMT programme and promotes cell differentiation' CELLULAR ONCOLOGY vol. 36, no. 4, 2013, pages 289 - 301, XP055380387**

**Description**

**Field of the invention**

The present invention relates to compositions for treating cancer.

**Background of the invention**

The use of proteases in treating cancer has been suggested for some time. Initially fresh pancreatic enzyme extracts was contemplated as a possible cancer therapy and some successful experiments were conducted with Jersen's mouse sarcoma model. After injecting the mouse with the protease enzyme trypsin, a regression of tumours was observed. The results obtained produced great interest, and crude enzyme extracts prepared from sheep pancreas were used to treat human cancer patients to reduce tumour progression and prolong survival time.

The use of proenzymes (inactive precursor form of enzymes) has been used to try to overcome problems encountered with the oral administration of enzymes with mixed results. A proenzyme mixture including trypsinogen, which is the proenzyme form of the serine protease inhibitor trypsin, has been shown to be useful in treating carcinomas and believed to be selectively activated at the surface of tumour cells. The mechanism of action of trypsin is believed to occur by way of proteolysis of the tumour cells. A composition including chymotrypsinogen and trypsinogen in a weight ratio range between 4:1 to 8:1 has been shown to be effective in assays for cancer, including pancreatic cancer and colon cancer (WO 2011/047434).

Novak et al. (Anticancer Research vol. 25, 2005, pp. 1157-1177) describe a mixture of trypsinogen and chymotrypsinogen at a 1:1 weight ratio, combined with amylase.

However, there exists a need to provide new or improved cancer treatments.

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

**Summary of the invention**

The present invention provides a pharmaceutical composition for use in the treatment of cancer, the composition comprising chymotrypsinogen, trypsinogen and a pharmaceutically acceptable diluent, excipient or carrier, wherein chymotrypsinogen is administered in an amount of equal to, or greater than, 3.5 mg/kg and no more than 41 mg/kg, and trypsinogen is administered in an amount of equal to, or greater than, 0.6 mg/kg and no more than 7 mg/kg.

In any aspect of the invention, the composition for use in treating cancer in a human comprises or consists of chymotrypsinogen and trypsinogen, wherein the amount of chymotrypsinogen to be administered is equal to or greater than 3.5mg/kg, 10mg/kg, 20mg/kg or 40 mg/kg, and no more than 41 mg/kg.

In any aspect of the invention, the composition for use in treating cancer in a human comprises or consists of chymotrypsinogen and trypsinogen, wherein the amount of trypsinogen to be administered is equal to or greater than 0.6mg/kg, 1.5mg/kg, 3mg/kg or 6mg/kg and no more than 7 mg/kg.

In one embodiment, the only active anti-tumour ingredients present in the composition are chymotrypsinogen and trypsinogen.

The invention provides a pharmaceutical composition for use in treating pancreatic cancer in a subject, the composition comprising chymotrypsinogen, trypsinogen and a pharmaceutically acceptable diluent, excipient or carrier, wherein the composition is adapted to administer chymotrypsinogen in an amount of equal to or greater than 5 mg/kg, preferably greater than 13mg/kg, and no more than 41 mg/kg, and trypsinogen in an amount of equal to or greater than 1.5mg/kg, preferably greater than 2mg/kg, and no more than 7 mg/kg.

The invention provides a pharmaceutical composition for use in treating ovarian cancer in a subject, the composition comprising chymotrypsinogen, trypsinogen and a pharmaceutically acceptable diluent, excipient or carrier, wherein the composition is adapted to administer chymotrypsinogen in an amount of equal to or greater than 4 mg/kg, and no more than 41 mg/kg, and trypsinogen in an amount of equal to or greater than 0.7 mg/kg, and no more than 7 mg/kg.

The present document further describes a method of treating cancer in a subject comprising administering chymotrypsinogen in a single dose or in multiple doses.

Preferably, the cancer is pancreatic cancer or ovarian cancer. Even more preferably, the pancreatic cancer is an adenocarcinoma.

The present document describes a method of treating cancer in a human comprising administering chymotrypsinogen in an amount of 40mg/kg and trypsinogen in an amount of 7mg/kg, thereby treating cancer. Preferably, the cancer is pancreatic cancer. Even more preferably, the pancreatic cancer is an adenocarcinoma.

The present document describes a method of treating cancer in a human comprising administering chymotrypsinogen in an amount of 4mg/kg or 13mg/kg and trypsinogen in an amount of 0.7mg/kg or 2mg/kg, thereby treating cancer. Preferably, the cancer is ovarian cancer.

In any aspect of the invention, the administration of the amount of trypsinogen and chymotrypsinogen does not result in any clinically observable adverse event in the subject 1 week after administration, 1 day after administration, or preferably 1 hour after administration. The clinically observable adverse event may be any one or more of weight loss, reddening at site of injection and behavioural changes, or any other event described herein, particularly the Examples.

Chymotrypsinogen and trypsinogen can be administered in a weight ratio in the range of at or about 4 : 1 to at or about 8 : 1, at or about 5 : 1 to at or about 7 : 1, or at about 6 : 1.

The present document also describes a kit for treating cancer comprising at least one dosage unit, wherein the dosage unit comprises chymotrypsinogen, trypsinogen and a pharmaceutically acceptable diluent, excipient or carrier, wherein the dosage unit is adapted to administer chymotrypsinogen and trypsinogen equal to, or greater than, any amount or mg/kg value described herein, including in Tables 1, 4, 8 and 10, particularly Example 5.

Optionally the kit also includes written instructions directing the user to administer a dosage unit of chymotrypsinogen in an amount as described herein, particularly Example 5.

The pharmaceutical compositions for use according to the invention are useful for treating cancers and metastatic carcinomas including pancreatic cancer, oesophageal cancer, colon cancer, bowel cancer, prostate cancer, ovarian cancer, brain cancer, stomach cancer, breast cancer, liver cancer, malignant melanoma or lung cancer. Preferably, the cancer is pancreatic cancer, colon cancer or ovarian cancer. More preferably, the cancer is pancreatic cancer.

In any aspect of a use of the invention, the use may further comprise the step of identifying a subject having, or at risk of developing, cancer. Preferably, the cancer is any one described herein.

In any aspect of the invention, the composition does not contain or the use does not administer amylase.

In any aspect, embodiment or form of the invention described herein the amount of chymotrypsinogen administered may be greater than, or equal to, 3.5mg/kg, 5mg/kg, 15mg/kg, 20mg/kg, or 40mg/kg. In any aspect, embodiment or form of the invention described herein the amount of trypsinogen administered may be greater than, or equal to, 0.6 mg/kg, 0.8mg/kg, 2mg/kg, 2.5mg/kg, 3mg/kg, or 5mg/kg,.

In any composition of the invention above, the composition may be adapted to administer the relevant mg, or mg/kg, of chymotrypsinogen and trypsinogen to the subject.

The invention also provides, any aspect of the invention above including values are 'about' the stated value above. For example, a further aspect of the invention is any of the aspects above where reference to 500mg/kg is about 500mg/kg. This is contemplated for all aspects or embodiments of the invention and for all values.

The invention provides a composition for use in treating cancer in a subject comprising chymotrypsinogen and trypsinogen wherein the weight ratio of chymotrypsinogen : trypsinogen is greater than 8:1 (i.e. 8 or more : 1). Preferably, the weight ratio is greater than 10:1. Preferably, the weight ratio is 10:1. The weight ratio may be 8:1, 10:1 or between 8: 1 and 10:1. In any of the embodiments, the composition preferably does not contain amylase, i.e. it comprises administration of an amylase-free composition.

Preferably, the cancer is any one or more of pancreatic cancer, oesophageal cancer, colon cancer, bowel cancer, prostate cancer, ovarian cancer, stomach cancer, breast cancer, liver cancer, malignant melanoma, fibrosarcoma or lung cancer. Preferably, the cancer is ovarian, melanoma, brain, prostate, colorectal, liver or lung. Preferably, the cancer is pancreatic cancer, colon cancer or ovarian cancer. More preferably, the cancer is pancreatic cancer.

In any aspect of the invention, chymotrypsinogen and trypsinogen are administered intravenously, subcutaneously or intramuscularly.

In any aspect of the invention, chymotrypsinogen and trypsinogen may be administered simultaneously or sequentially.

As used herein, except where the context requires otherwise, the term "comprise" and variations of the term, such as "comprising", "comprises" and "comprised", are not intended to exclude further additives, components, integers or steps.

Further aspects of the present invention and further embodiments of the aspects described in the preceding paragraphs will become apparent from the following description, given by way of example and with reference to the accompanying drawings.

## Brief description of the drawings

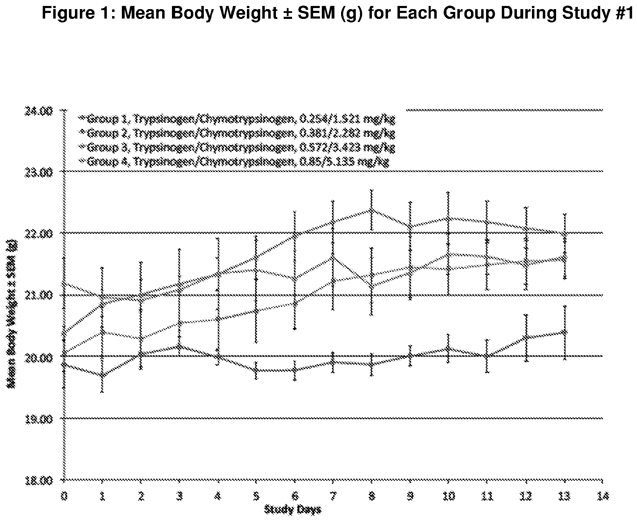

**Figure 1:** Mean Body Weight $\pm$ SEM (g) for Each Group During Study # 1. Treatments were administered as single i.v. injection, once daily for seven consecutive days. Treatment was initiated to each group in a staggered manner, beginning with the lowest dose and then increasing the dose on each subsequent day. For all data calculations and presentation, the first day of treatment for each group is designated as Study Day 0.

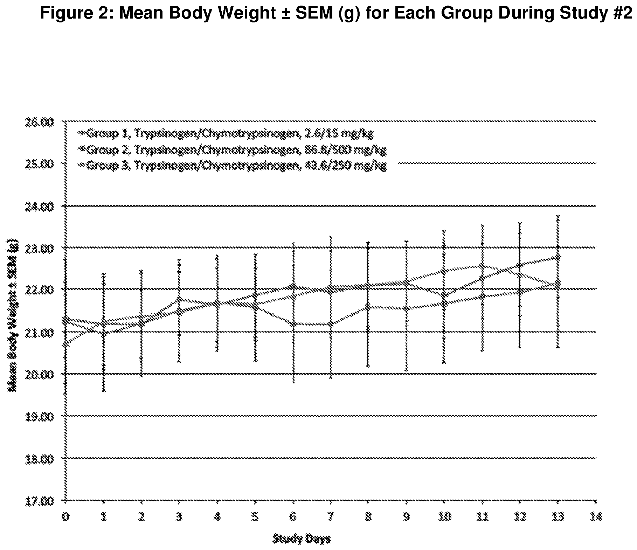

**Figure 2:** Mean Body Weight $\pm$ SEM (g) for Each Group During Study #2. Treatments were administered as single i.v. injection, once daily for seven consecutive days. Treatment was initiated to each group in a staggered manner, beginning with the lowest dose on the first day and then increasing the dose to Trypsinogen/Chymotrypsinogen A at 86.8/500 mg/kg on the second day. Treatment with Trypsinogen/Chymotrypsinogen A at 43.4/250 mg/kg commenced on the third day. For all data calculations and presentation, the first day of treatment for each group is designated as Study Day 0.

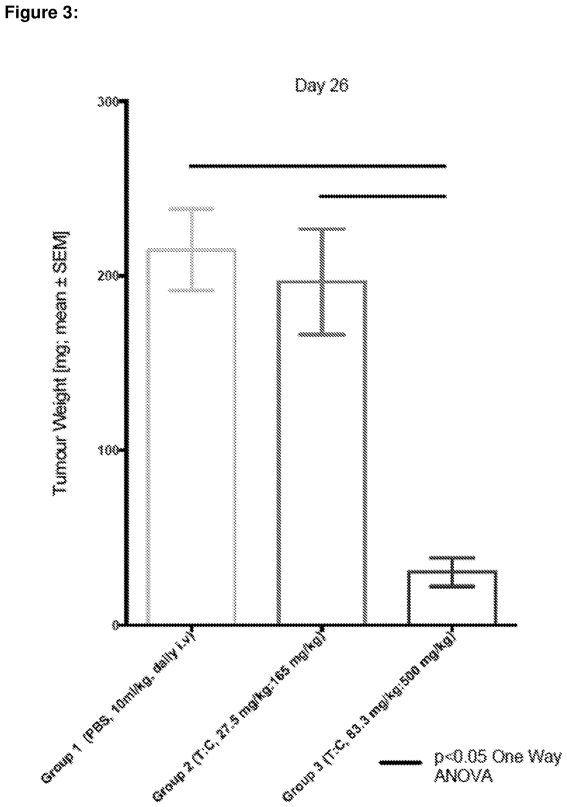

**Figure 3:** Results of mean tumour weight $\pm$ SEM (mg) of PBS control mice (Group 1, n=8) and mice receiving T:C, 27.5 mg/kg:165 mg/kg (Group 2, n=9) or T:C, 83.3 mg/kg:500 mg/kg (Group 3, n=10) in study described in Example 3 performed to assess anti-tumour efficacy of Trypsinogen and Chymotrypsinogen A, administered in combination against Pan02 mouse pancreatic cancer cells, orthotopically inoculated in female C57BL/6 mice. Treatments were administered as single i.v. injection, one daily on Study Days 0 to 25. Animals were terminated on Study Day 26.

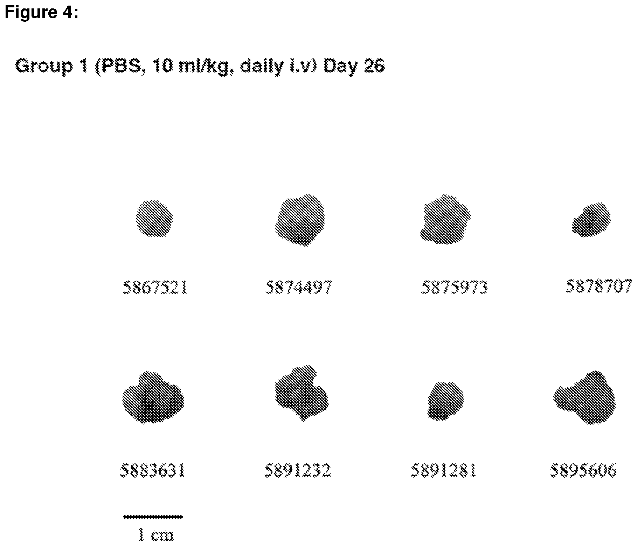

**Figure 4:** Images of excised tumours from mice in Group 1 (PBS, 10 ml/kg, daily i.v) Day 26 in study described in Example 3 performed to assess anti-tumour efficacy of Trypsinogen and Chymotrypsinogen A, administered in combination against Pan02 mouse pancreatic cancer cells, orthotopically inoculated in female C57BL/6 mice.

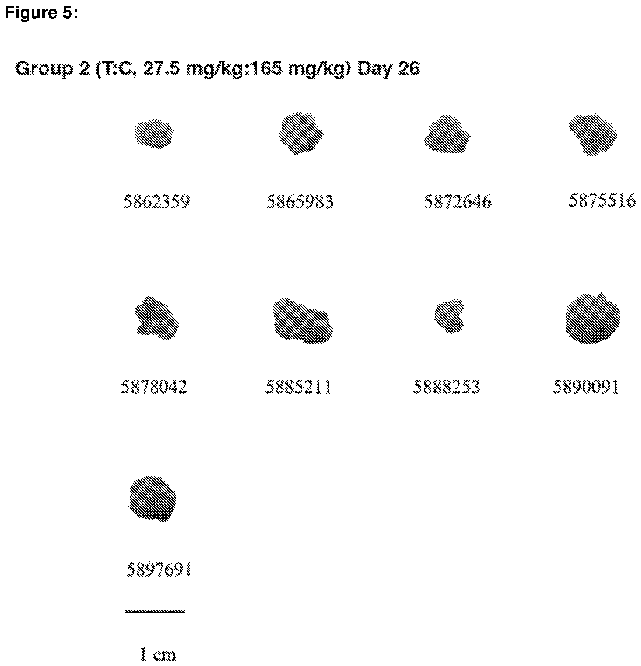

**Figure 5:** Images of excised tumours from mice in Group 2 (T:C, 27.5 mg/kg: 165 mg/kg) Day 26 in study described in Example 3 performed to assess anti-tumour efficacy of Trypsinogen and Chymotrypsinogen A, administered in combination against Pan02 mouse pancreatic cancer cells, orthotopically inoculated in female C57BL/6 mice.

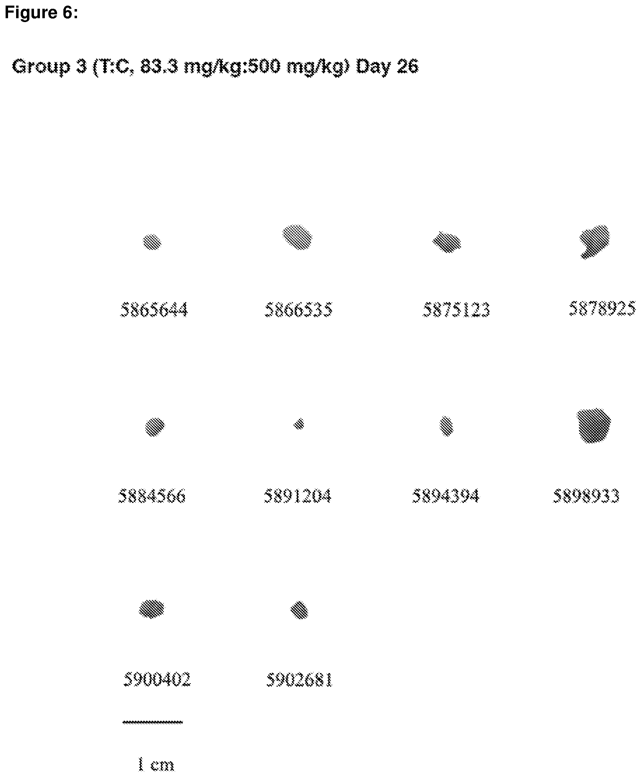

**Figure 6:** Images of excised tumours from mice in Group 3 (T:C, 83.3 mg/kg:500 mg/kg) Day 26 in study described in Example 3 performed to assess anti-tumour efficacy of Trypsinogen and Chymotrypsinogen A, administered in combination against Pan02 mouse pancreatic cancer cells, orthotopically inoculated in female C57BL/6 mice.

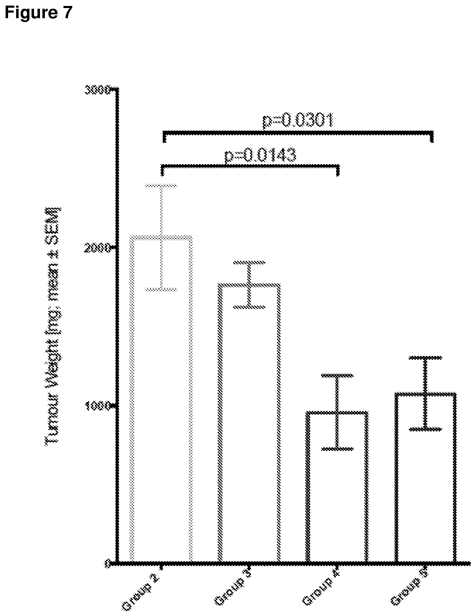

**Figure 7:** Mean Tumour Weight $\pm$ SEM (g) in Group 2: Vehicle Control (PBS). Group 3: Trypsinogen/Chymotrypsinogen A, 83.3/500 mg/kg. Group 4: Trypsinogen/Chymotrypsinogen A, 27.5/165 mg/kg. Group 5: Trypsinogen/Chymotrypsinogen A, 9.1/54 mg/kg, in study described in Example 4 performed to assess anti-tumour efficacy of Trypsinogen and Chymotrypsinogen A, administered in combination, against A2780 human ovarian cancer cells orthotopically inoculated in female Athymic Nude-*Foxn1*$^{nu}$ mice. Treatments were administered as a single i.v. injection, once daily (Study Days 0 to 20). These animals were terminated on Study Day 21.

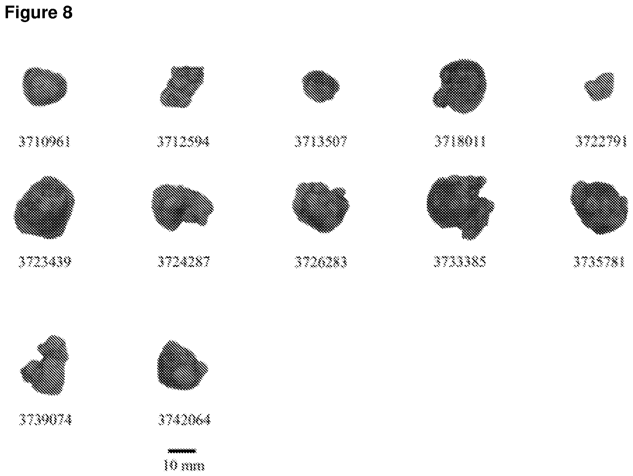

**Figure 8:** Images of excised tumours at study termination for Group 2: Vehicle Control (PBS) in study described in Example 4 performed to assess anti-tumour efficacy of Trypsinogen and Chymotrypsinogen A, administered in combination, against A2780 human ovarian cancer cells orthotopically inoculated in female Athymic Nude-*Foxn1*$^{nu}$ mice.

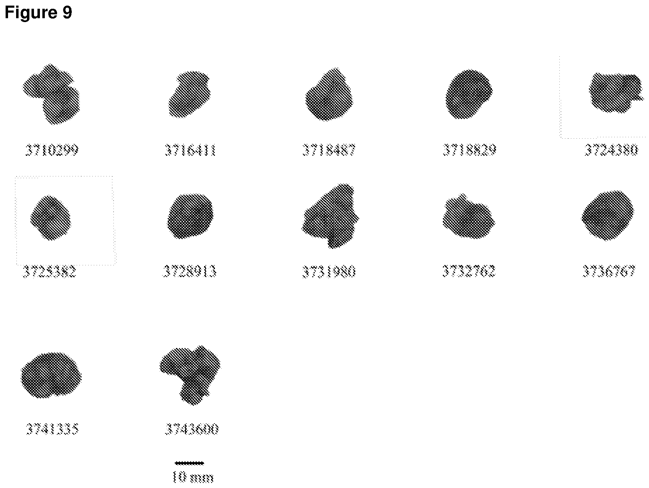

**Figure 9:** Images of excised tumours at study termination for Group 3: Trypsinogen/Chymotrypsinogen A, 83.3/500 mg/kg in study described in Example 4 performed to assess anti-tumour efficacy of Trypsinogen and Chymotrypsinogen A, administered in combination, against A2780 human ovarian cancer cells orthotopically inoculated in female Athymic Nude-*Foxn1*$^{nu}$ mice.

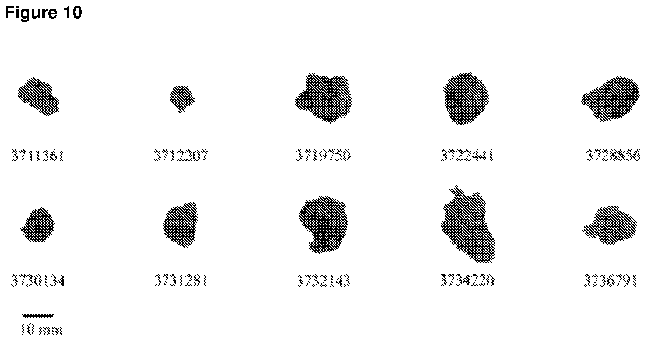

**Figure 10:** Images of excised tumours at study termination for Group 4: Trypsinogen/Chymotrypsinogen A, 27.5/165 mg/kg in study described in Example 4 performed to assess anti-tumour efficacy of Trypsinogen and Chymotrypsinogen A, administered in combination, against A2780 human ovarian cancer cells orthotopically inoculated in female Athymic Nude-*Foxn1*$^{nu}$ mice.

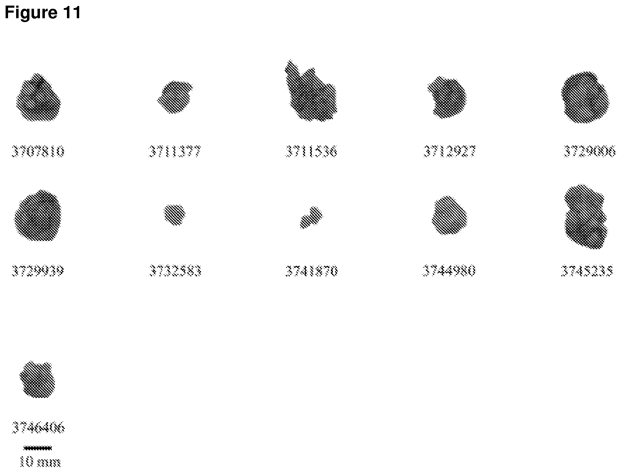

**Figure 11:** Images of excised tumours at study termination for Group 5: Trypsinogen/Chymotrypsinogen A, 9.1/54 mg/kg in study described in Example 4 performed to assess anti-tumour efficacy of Trypsinogen and Chymotrypsinogen A, administered in combination, against A2780 human ovarian cancer cells orthotopically inoculated in female Athymic Nude-*Foxn1*$^{nu}$ mice.

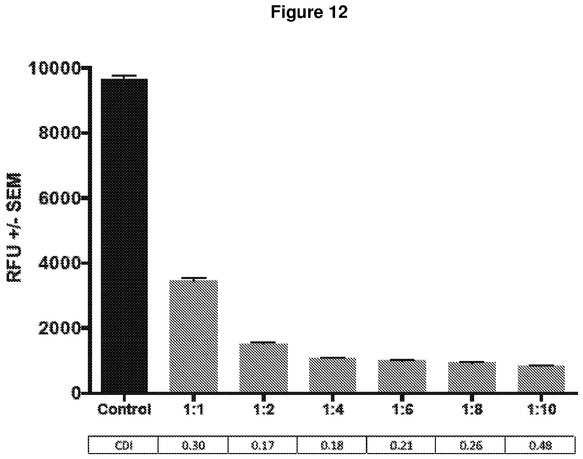

**Figure 12:** The effect of Trypsinogen and Chymotrypsinogen alone or in combination on the growth of A2780 human ovary tumour cells. Combination assays of Trypsinogen and Chymotrypsinogen were performed at ratios of 1:1, 1:2, 1:4, 1:6, 1:8 and 1:10 based on the previously determined $IC_{50}$ of Trypsinogen (3.174mg/ml). CDI values were calculated as described.

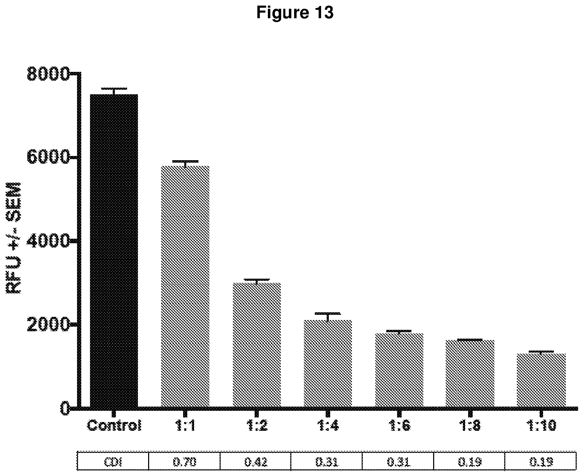

**Figure 13:** The effect of Trypsinogen and Chymotrypsinogen alone or in combination on the growth of C8161.9 human melanoma cells. Combination assays of Trypsinogen and Chymotrypsinogen were performed at ratios of 1:1, 1:2, 1:4, 1:6, 1:8 and 1:10 based on the previously determined $IC_{50}$ of Trypsinogen (3.917mg/ml). CDI values were calculated as described.

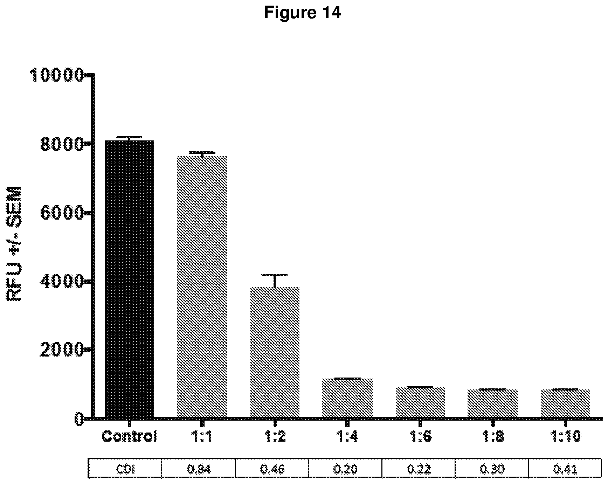

**Figure 14:** The effect of Trypsinogen and Chymotrypsinogen alone or in combination on the growth of DAOY human brain tumour cells. Combination assays of Trypsinogen and Chymotrypsinogen were performed at ratios of 1:1, 1:2, 1:4, 1:6, 1:8 and 1:10 based on the previously determined $IC_{50}$ of Trypsinogen (2.654mg/ml). CDI values were calculated as described.

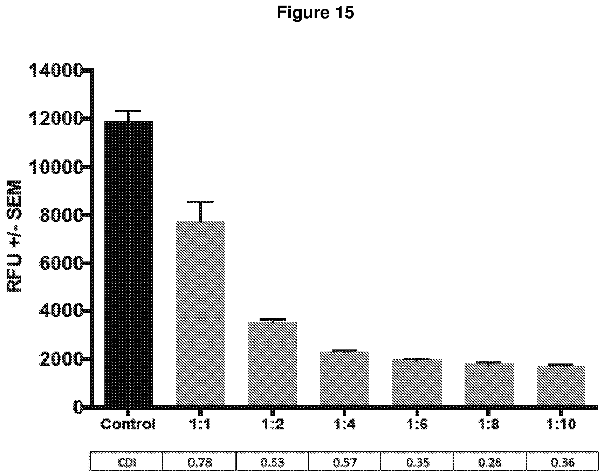

**Figure 15:** The effect of Trypsinogen and Chymotrypsinogen alone or in combination on the growth of DU145 human prostate tumour cells. Combination assays of Trypsinogen and Chymotrypsinogen were performed at ratios of 1:1, 1:2, 1:4, 1:6, 1:8 and 1:10 based on the previously determined $IC_{50}$ of Trypsinogen (3.843mg/ml). CDI values were calculated as described.

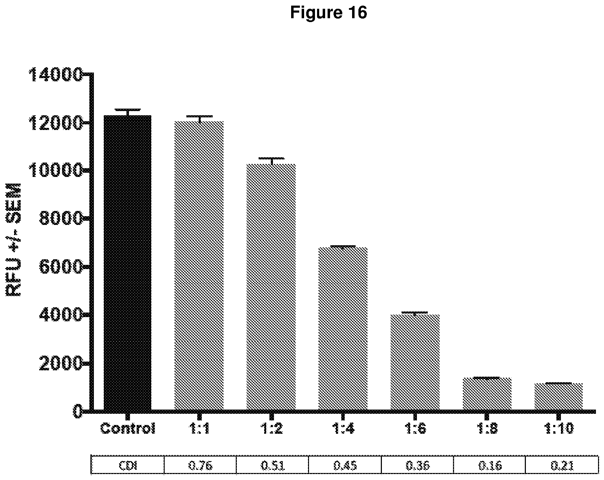

**Figure 16:** The effect of Trypsinogen and Chymotrypsinogen alone or in combination on the growth of HCT 116 human colorectal tumour cells. Combination assays of Trypsinogen and Chymotrypsinogen were performed at ratios of 1:1, 1:2, 1:4, 1:6, 1:8 and 1:10 based on the previously determined $IC_{50}$ of Trypsinogen (16.43mg/ml). CDI values were calculated as described.

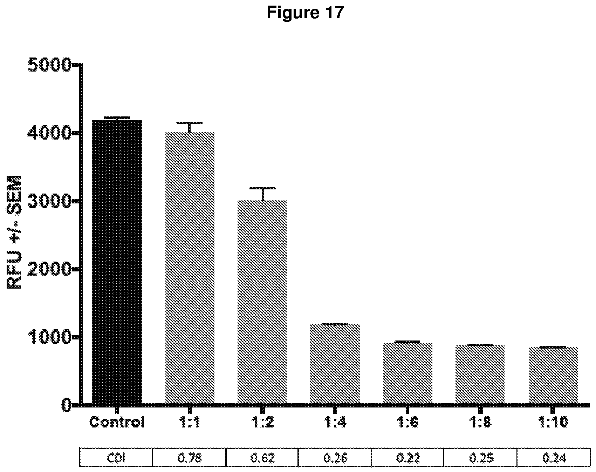

**Figure 17:** The effect of Trypsinogen and Chymotrypsinogen alone or in combination on the growth of Hep3B2.1-7 human liver tumour cells. Combination assays of Trypsinogen and Chymotrypsinogen were performed at ratios of 1:1, 1:2, 1:4, 1:6, 1:8 and 1:10 based on the previously determined $IC_{50}$ of Trypsinogen (2.483mg/ml). CDI values were calculated as described.

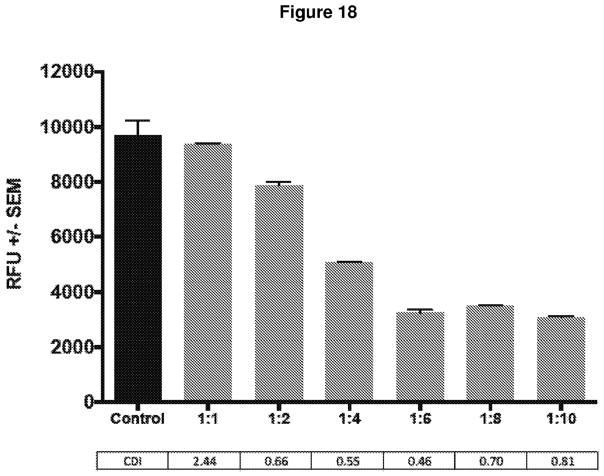

**Figure 18:** The effect of Trypsinogen and Chymotrypsinogen alone or in combination on the growth of HT-29 human colorectal tumour cells. Combination assays of Trypsinogen and Chymotrypsinogen were performed at ratios of 1:1, 1:2, 1:4, 1:6, 1:8 and 1:10 based on the previously determined $IC_{50}$ of Trypsinogen (15.12mg/ml). CDI values were calculated as described.

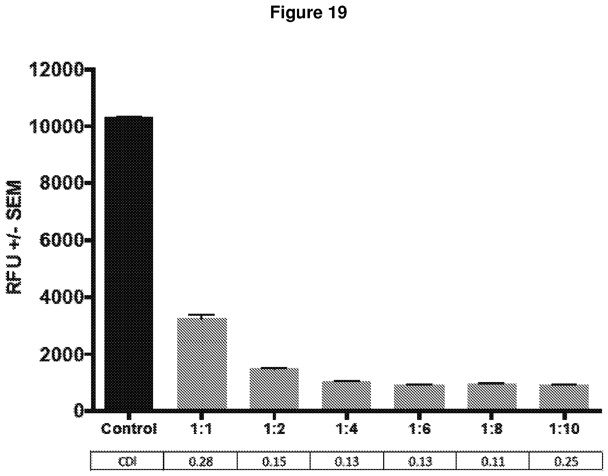

**Figure 19:** The effect of Trypsinogen and Chymotrypsinogen alone or in combination on the growth of HuH-7 human liver tumour cells. Combination assays of Trypsinogen and Chymotrypsinogen were performed at ratios of 1:1, 1:2, 1:4, 1:6, 1:8 and 1:10 based on the previously determined $IC_{50}$ of Trypsinogen (3.934mg/ml). CDI values were calculated as described.

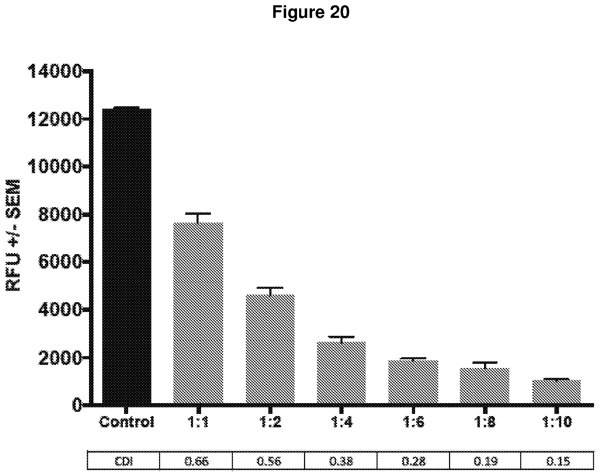

**Figure 20:** The effect of Trypsinogen and Chymotrypsinogen alone or in combination on the growth of NCI-H460 human lung tumour cells. Combination assays of Trypsinogen and Chymotrypsinogen were performed at ratios of 1:1, 1:2, 1:4, 1:6, 1:8 and 1:10 based on the previously determined $IC_{50}$ of Trypsinogen (4.028mg/ml). CDI values were calculated as described.

## Detailed description of the embodiments

Reference will now be made in detail to certain embodiments of the invention. While the invention will be described in conjunction with the embodiments, it will be understood that the intention is not to limit the invention to those embodiments. On the contrary, the invention is intended to cover all alternatives, modifications, and equivalents, which may be included within the scope of the present invention as defined by the claims.

For purposes of interpreting this specification, terms used in the singular will also include the plural and vice versa.

The present invention is based on the surprising finding that chymotrypsinogen and trypsinogen can be administered at amounts significantly higher than previously used. This could occur in a single administration, or over a period of a single day. Surprisingly, these significantly higher amounts of chymotrypsinogen and trypsinogen, even hundred fold higher amounts, can be administered without any significant adverse clinical events. In addition, these higher amounts are shown to reduce the weight of several tumour types *in vivo.* The present invention provides an important benefit in the treatment of cancer as a larger effective dose of chymotrypsinogen and trypsinogen can be safely administered than previously used thereby providing a greater therapeutic window.

Chymotrypsinogen (which may be abbreviated to 'C' herein) is a proenzyme form of the enzyme chymotrypsin, which preferentially cleaves proteins at the following amino acids: tyrosine, tryptophan, phenylalanine and leucine. Chymotrypsin may be referred to or includes chymotrypsin A, chymotrypsin B (including B1 and B2 forms), chymotrypsin C, a-chymarophth, avazyme, chymar, chymotest, enzeon, quimar, quimotrase, α-chymar, α-chymotrypsin A, α-chymotrypsin. Chymotrypsin C can be formed from pig chymotrypsinogen C or from cattle subunit II of procarboxypeptidase A, and preferentially cleaves proteins at the following amino acids: tyrosine, tryptophan, phenylalanine, leucine, methionine, glutamine, and asparagine. Chymotrypsinogen includes chymotrypsinogen B1 and chymotrypsinogen B2.

Trypsinogen (which may be abbreviated to 'T' herein) is a proenzyme form of trypsin, which preferentially cleaves proteins at arginine and lysine. Trypsin may be referred to or include α-trypsin, β-trypsin, cocoonase, parenzyme, parenzymol, tryptar, trypure, pseudotrypsin, tryptase, tripcellim, sperm receptor hydrolase β-trypsin can be formed from trypsinogen by cleavage of one peptide bond. Further peptide bond cleavages produce α and other iso-forms. Multiple cationic and anionic trypsins can be isolated from the pancreas of many vertebrates and from lower species including crayfish, insects (cocoonase) and microorganisms (Streptomyces griseus). In normal processes during digestion, inactive trypsinogen is activated by enteropeptidase present in intestinal mucosa to form the enzyme trypsin, which being a serine protease then acts to cleave the peptide bonds on the carboxyl side of basic amino acids/proteins.

The trypsinogen and chymotrypsinogen used in any aspect of the invention may be isolated, purified, substantially purified, recombinant or synthetic.

The proenzymes trypsinogen and chymotrypsinogen may be precursors of the enzymes selected from chymotrypsin classes 3.4.21.1 or 3.4.21.2 or trypsin from class 3.4.21.4, or selected from any other suitable source (classes grouped according to the classification of the Nomenclature Committee of the International Union of Biochemistry and Molecular Biology). These enzymes are commercially available and may be of human, bovine or porcine origin.

As used herein, "any other amount as described herein" or "any mg/kg value described herein" includes any amount or mg/kg value described in any of the Examples including in Tables 1, 4, 8 and 10.

The present invention includes human conversions for all amounts in mg/kg referred to herein based on human body weights of 50, 60, 70, 80, 90, 100 or more kg and body surface area of 1.1, 1.2, 1.3, 1.4, 1.5, 1.6, 1.7, 1.8 or more $m^2$. Specifically, amounts per kg for human administration, and methods for calculation, are referred to in Example 5.

As mentioned, the proenzyme form essentially provides an inactivated form of the enzyme that becomes activated *in situ* (e.g *in vivo* or *in vitro* activation). For example, activation of the proenzyme (conversion of proenzyme to active enzyme) may occur on contact with the surface of the tumour cell. It is believed that the proenzymes trypsinogen and chymotrypsinogen are selectively activated into the enzymes trypsin and chymotrypsin on contact with tumour cells and not on contact with healthy cells. The use of proenzymes reduces problems associated with providing, *in situ,* an active enzyme, such as undesirable reactions or inactivation of the enzyme before reaching an intended target of a tumour cell.

In relation to tumour cells, protease enzymes can act to break down the cell wall of malignant cells by cleaving the amide bonds present in peptide chains of the cell walls (proteolysis). It is also understood that protease inhibitors, which are present in non-malignant cells and inhibit or reduce the effect of enzymes in breaking down cell walls, are absent in malignant tumour cells. In addition to providing proteolytic activity, the protease proenzymes can upregulate the expression of β-catenin and E-cadherin in tumour cells. Cell-to-cell adhesion is facilitated by complex formation or bonding between β-catenin and E-cadherin at the cell surface, and therefore increased expression of β-catenin and E-cadherin leads to enhanced cell-to-cell adhesion and thereby reducing metastasis of tumour cells. The protease proenzymes may also provide other cellular activity such as increased immunorecognition or differentiation.

A significant adverse event, experience or reaction is any untoward medical occurrence that at any dose: results in death, is life-threatening (places the subject at immediate risk of death), requires in subject/subject hospitalization or prolongation of existing hospitalization, results in persistent or significant disability, incapacity, or is a congenital anomaly/birth defect.

A clinically observable adverse event is any untoward medical occurrence in a subject or clinical investigation subject administered a pharmaceutical product. A clinically observable adverse event may include any one of the events or observations described herein, particularly in the Examples. Typically, the period of observation is about 1 week after administration, about 1 day after administration, or preferably about 1 hour after administration. The period of observation may be the time between administration of doses.

"Treating" or "treatment" refers to both therapeutic treatment and prophylactic or preventative measures, wherein the aim is to prevent, ameliorate, reduce or slow down (lessen) cancer or the spread (metastasis) thereof.

In any method of the present disclosure, one or more of the following effects may be observed: reduction in the reoccurrence of malignant tumours, reduction in metastasis of malignant tumours, reduction in number or size of tumours, differentiation of tumour cells, expression of β-catenin and E-cadherin in malignant tumours to facilitate cell-to-cell adhesion and reduction in metastasis, reduction in tumour cells ability to prevent immunorecognition.

"Preventing", "prevention", "preventative" or "prophylactic" refers to keeping from occurring, or to hinder, defend from, or protect from the occurrence of a condition, disease, disorder, or phenotype, including an abnormality or symptom. A subject in need of prevention may be prone to develop the condition.

The term "ameliorate" or "amelioration" refers to a decrease, reduction or elimination of a condition, disease, disorder, or phenotype, including an abnormality or symptom. A subject in need of treatment may already have the condition, or may be prone to have the condition or may be one in whom the condition is to be prevented.

The "subject" includes a mammal. The mammal may be a human, or may be a domestic, zoo, or companion animal. While it is particularly contemplated that the methods of the invention are suitable for medical treatment of humans, they are also applicable to veterinary treatment, including treatment of companion animals such as dogs and cats, and domestic animals such as horses, cattle and sheep, or zoo animals such as felids, canids, bovids, and ungulates. A subject may be afflicted with cancer or other disorder, or may not be afflicted with cancer or other disorder (i.e., free of detectable disease).

The typical body weight of a human subject may be greater than, or equal to, 60, 65, 70, 75, 80, 85, 90, 95, 100, 105 or 110kg.

The term "therapeutically effective amount" refers to an amount of composition, or agent or compound in the composition, capable of treating, preventing or ameliorating cancer or the spread (metastasis) thereof. A therapeutically effective amount may be determined empirically and in a routine manner in relation to treating cancer, and will result in increased life expectancy.

As used herein "adapted to administer" refers to a composition that has the capacity to administer trypsinogen and chymotrypsinogen at the specified amount or concentration in a single dose or multiple doses.

As described herein, methods include treatment of neoplasms and related conditions, cancers, tumours, malignant and metastatic conditions. Tissues and organs associated with solid tumours and metastases which can be treated with a method or pharmaceutical composition of the invention include, but are not limited to, biliary tract, bladder, blood, brain, breast, cervix, colon, endometrium, esophagus, head, neck, kidney, larynx, liver, lung, medulla, melanin, ovarian, pancreas, prostate, rectum, renal, retina, skin, stomach, testes, thyroid, urinary tract, and uterus. The cancer may be a carcinoma and developed in an epithelium or from epithelial cells. The cancer may be a sarcoma and developed in connective tissue. The brain cancer may be glioblastoma.

The pharmaceutical compositions for use of the invention are useful for treating cancers and metastatic carcinomas of the following types: pancreatic cancer, oesophageal cancer, colon cancer, bowel cancer, prostate cancer, ovarian cancer, stomach cancer, breast cancer, malignant melanoma or lung cancer. Preferably, the cancer is pancreatic cancer, colon cancer or ovarian cancer. More preferably, the cancer is pancreatic cancer. The metastatic carcinoma may have low, moderate or high metastatic potential.

The pharmaceutical compositions may provide a multiple effect approach to treating cancer, for example by increasing in tumour cells apoptosis, cell-to-cell adhesion, differentiation and immunogenicity (targeting and removal by immune system). It is therefore beneficial to conduct treatment in the absence of any other treatments that may suppress or harm the immune system.

Compositions for use of the invention can be supplemented by other conventional anti-cancer therapeutic approaches directed to treatment or prevention of proliferative disorders (e.g., tumour). For example, such methods can be used in prophylactic cancer prevention, prevention of cancer recurrence and metastases after surgery, and as an adjuvant of other conventional cancer therapy.

The pharmaceutical compositions for use of the invention may be formulated, for example, by employing conventional solid or liquid vehicles or diluents, as well as pharmaceutical additives of a type appropriate to the mode of desired administration (for example, excipients, binders, preservatives, stabilizers, flavors, etc.) according to techniques such as those well known in the art of pharmaceutical formulation.

The pharmaceutical compositions for use of the invention may be administered by any suitable means, for example, orally, such as in the form of tablets, capsules, granules or powders; sublingually; buccally; parenterally, such as by subcutaneous, intravenous, intramuscular, or intracisternal injection or infusion techniques (e.g., as sterile injectable aqueous or non-aqueous solutions or suspensions); nasally such as by inhalation spray; topically, such as in the form of a cream or ointment; or rectally such as in the form of suppositories; in dosage unit formulations containing non-toxic, pharmaceutically acceptable vehicles or diluents. They may, for example, be administered in a form suitable for immediate release or extended release, for example, by the use of devices such as subcutaneous implants, encapsulated spheroids or osmotic pumps.

In addition to primates, such as humans, a variety of other mammals can be treated according to the methods of the tenth aspect. For instance, mammals including, but not limited to, cows, sheep, goats, horses, dogs, cats, guinea pigs, rats or other bovine, ovine, equine, canine, feline, rodent or murine species can be treated.

The term "pharmaceutically acceptable" as used herein means the carrier, diluent or excipient is not deleterious to the recipient thereof.

The terms "administration of and or "administering" should be understood to mean providing to an individual in need of treatment.

An individual in need of treatment may be one diagnosed with, or at risk of developing, any one of the cancers described herein.

The pharmaceutical compositions for use of the invention, and preparations or formulations thereof may be prepared by admixing together the components of the composition, namely chymotrypsinogen and trypsinogen. The admixing may be performed sequentially or simultaneously.

The pharmaceutical compositions for use of the invention may conveniently be presented in dosage unit form and may be prepared by any of the methods well known in the art of pharmacy. All methods include the step of bringing the active agents and protease proenzyme into association with the carrier which constitutes one or more accessory ingredients. In general, the pharmaceutical compositions are prepared by uniformly and intimately bringing the active agents and protease proenzymes into association with a liquid carrier or a finely divided solid carrier or both, and then, if necessary, shaping the product into the desired formulation. The active agents and protease proenzymes are provided in a dosage unit form in an amount sufficient to produce the desired effect upon the process or condition of diseases after single or repeated administration.

The pharmaceutical compositions for use of the invention may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the protease proenzyme and active agent of the first and second aspects in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated to form osmotic therapeutic tablets for control release.

Formulations for oral use may also be presented as hard gelatin capsules wherein the protease proenzyme and active agent of the first and second aspects are mixed with an inert solid diluent, for example, calcium carbonate, calcium phosphate or kaolin, or as soft gelatin capsules wherein the protease proenzyme and active agent of the first and second aspects are mixed with water or an oil medium, for example peanut oil, liquid paraffin, or olive oil.

Aqueous suspensions contain the active agent and protease proenzyme in admixture with excipients suitable for the manufacture of aqueous suspensions. Such excipients are suspending agents, for example sodium carboxymethylcellulose, methylcellulose, hydroxy-propylmethylcellulose, sodium alginate, polyvinyl-pyrrolidone, n-methyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active agent and protease proenzyme in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These may be preserved by the addition of an anti-oxidant such as ascorbic acid.

Dispersible powders and granules suitable for preparation of an aqueous suspension by the addition of water provide the protease proenzyme and active agent of the first and second aspects in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present.

The pharmaceutical compositions for use of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally- occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxy ethylene sorbitan monooleate. The emulsions may also contain sweetening and flavouring agents.

Syrups and elixirs may be formulated with sweetening agents, for example glycerol, propylene glycol, sorbitol or sucrose. They may also contain a demulcent, a preservative and flavoring and coloring agents.

The pharmaceutical compositions for use of invention may be in the form of a sterile injectable aqueous or oleagenous suspension. This suspension may be formulated according to the known art using those suitable dispersing or wetting agents and suspending agents which have been mentioned above. The pharmaceutical compositions of the first and second aspects may also be a sterile injectable solution or suspension in a non-toxic parenterally-acceptable diluent or solvent, for example as a solution in 1 ,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

In a particular embodiment, the pharmaceutical compositions for use of the invention are formulated as suppositories for rectal administration of the drug. These formulations can be prepared by mixing the protease proenzyme and active agent of the first and second aspects with a suitable non-irritating excipient which is solid at ordinary temperatures but liquid at the rectal temperature and will therefore melt in the rectum to release the drug. Such materials include cocoa butter and polyethylene glycols. Rectal administration may be used to elimate entero-hepatic first pass effect in the gastrointestinal tract related to oral administration of enzymes.

The pharmaceutical compositions for use of the invention, may also be formulated in liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolisable lipid capable of forming liposomes can be used. The liposome formulation may contain stabilisers, preservatives, excipients and the like. The preferred lipids are the phospholipids and phosphatidyl cholines, both natural and synthetic. Methods to form liposomes are known in the art.

The pharmaceutical compositions for use of the invention, may be included in a container, pack, or dispenser together with instructions for administration. The protease proenzymes and active agents, and optionally additional active agent, of the pharmaceutical composition may be provided as separated components in the container, pack, or dispenser, to be taken separately or together at the same or different time in a use or method of the invention described herein.

## Examples

### Example 1

A dosing study was completed to determine the maximum tolerated and feasible dose in which no adverse clinical events were observed, or in which any adverse clinical events were resolved by the subsequent dosing. The dosing study protocol is set out below.

Trypsinogen and Chymotrypsinogen A were dissolved in phosphate buffered saline (PBS) on each treatment day to give stock solutions of 1 mg/mL. Dosing solutions of the required concentrations of Trypsinogen/Chymotrypsinogen A were prepared by diluting the stock solutions together in PBS to achieve the desired final concentration of each.

For Study #1, dosing solutions of Trypsinogen/Chymotrypsinogen A at 0.0254/0.1521, 0.0381/0.2282, 0.0572/0.3423 and 0.0858/0.5135 mg/mL were prepared.

For the study, treatments were administered to animals in each group once daily for seven consecutive days in a dosing volume of 10 mL/kg. The volume of dosing solution administered to each animal was calculated and adjusted based on individual body weight measured immediately prior to dosing.

For Study #1 mice were randomised by body weight into four groups of four mice. The dosing regimen used for the study is summarised in Table 1. Trypsinogen and Chymotrypsinogen A were administered in combination as a single intravenous tail vein injection (i.v.) at doses of 10 mL/Kg. The final concentration of Trypsinogen and Chymotrypsinogen for each group were 0.254/1.521, 0.381/2.282, 0.572/3.423 and 0.858/5.135 mg/kg (Groups 1, 2, 3 and 4, respectively).

Treatment for each group in Study #1 was initiated in a staggered manner, beginning with the lowest dose of Trypsinogen/Chymotrypsinogen A, and then increasing the dose on each subsequent study day. For all data calculations and presentation, the first day of treatment for each group is designated as Study Day 0.

On the first day of each study, one mouse in Group 1 was treated with Trypsinogen/Chymotrypsinogen A at the lowest dose (0.254/1.521 mg/kg for Study #1). As there were no prolonged severe adverse effects within two hours of treatment, the remaining three mice in each of these groups were similarly treated.

As there were no prolonged severe adverse effects within 24 hours of initial treatment in Group 1 mice, one mouse in Group 2 was treated with Trypsinogen/Chymotrypsinogen A at 0.381/2.282 mg/kg for Study #1. As there were no visible signs of toxicity within two hours of this treatment, the remaining mice in each of these groups received the same treatment.

Treatment continued in this manner for the remaining two doses of Trypsinogen/Chymotrypsinogen A for Study #1 (0.572/3.423 and 0.858/5.135 mg/kg) to animals in Groups 3 and 4, respectively.

All animals in each group in each study received seven consecutive daily treatments.

**Mortality**          Mortality checks were performed once daily in the morning during the study.

**Clinical Observations**   Animals were checked for clinical signs (such as ill health and behavioural changes) twice daily during the study.

**Body Weights**   Body weights were recorded for all animals on the first treatment day, then daily during the treatment period and for seven days following final treatment.

**Clinical Observations:**

| | |
|---|---|
| *From Distance* | |
| Gait | Normal or Abnormal |
| Piloerection | Normal or Abnormal |
| Type of breathing | Normal or Abnormal |
| *On Handling* | |
| Inquisitive & alert | Normal or Abnormal |
| Body temperature | Normal or Abnormal |
| Skin Tone | Normal or Abnormal |
| Eye condition (pale, sunken) | Normal or Abnormal |
| Dehydration | Normal or Abnormal |
| Diarrhoea | Normal or Abnormal |
| Abdomen (distended/swollen) | Normal or Abnormal |
| Other signs of adverse reaction to drug or ill health | Normal or Abnormal |
| Signs previously observed, e.g. in a previous study or with other species | Normal or Abnormal |
| Skin Reddening/Irritation e.g. Radiotherapy side effects | Normal or Abnormal |
| Body condition | BC1, BC2, BC3, BC4, BC5 |

**Assessment Criteria for Clinical Observations:**

**Gait:** Limping, Paresis, Paralysis = Abnormal

**Body Temperature:** Warm or cold = Abnormal

**Type of breathing:** Rapid; Shallow or Laboured = Abnormal

**Diarrhoea:** Loose faeces on floor of the cage, pools of faeces on floor of the cage or running out on handling = Abnormal

**Body Condition** (Ref: Ullman-Cullere & Foltz. Lab. Animal Science 1999; 49:319): BC1 = Animal is emaciated; BC2 = Animal is underconditioned; BC3 = Animal is well-conditioned; BC4 = Animal is over-conditioned; BC5 = Animal is obese

**Clinical Observations and Adverse Events for Study #1**

No adverse clinical signs were observed in any animals during the study.

**Body Weight Changes**

There was mean body weight gain over the course of the study (Study Days 0 to 13) for each group (2.59%, 7.48%, 7.97% and 2.03% of initial weight for animals treated with Trypsinogen and Chymotrypsinogen A at 0.254/1.521, 0.381/2.282, 0.572/3.423 and 0.858/5.135 mg/kg; Groups 1, 2, 3 and 4, respectively) (Table 2 and Figure 1). No animals lost body weight in excess of 15% of initial weight (Table 3).

**Example 2**

Surprisingly, the amounts of Trypsinogen and Chymotrypsinogen A in Study #1 were well tolerated as shown from the results in Example 2 above. This was unexpected given these amounts were significantly above amounts previously used. In view of these results, a further study, Study #2, was performed using amounts of Trypsinogen and Chymotrypsinogen A, in combination, at even higher than ever previously used amounts.

For Study #2, a stock solutions of Trypsinogen and Chymotrypsinogen A were prepared at 5 and 6 mg/mL respectively. The stocks solutions were further diluted in PBS to prepare the dosing solutions of Trypsinogen/Chymotrypsinogen A for Group 1 of 0.256/1.5 mg/ml. For Groups 2 and 3, a stock solutions of Trypsinogen and Chymotrypsinogen A were prepared at 30 and 100 mg/mL respectively.

The stocks solutions were further diluted in PBS to prepare the dosing solutions of Trypsinogen/Chymotrypsinogen A for Group 2 of 8.68/50 mg/ml and for Group 3 of 4.34/25 mg/ml. It was noted that at a concentration of 50 mg/ml Chymotrypsinogen A was almost at the limit of viscosity for tail vein injection.

For Study #2 12 mice were randomised by body weight into three groups of four mice. The dosing regimen used for the study is summarised in Table 4. Trypsinogen and Chymotrypsinogen A were administered in combination as a single intravenous tail vein injection (i.v.) at doses of 10 mL/Kg. The final concentration of Trypsinogen and Chymotrypsinogen for each group were 2.6/15, 86.8/500 and 43.4/250 mg/kg (Groups 1, 2 and 3, respectively)

Treatment for each group in Study #2 was initiated in a staggered manner, beginning with the lowest dose of Trypsinogen/Chymotrypsinogen A, and then increasing the dose on each subsequent study day. For all data calculations and presentation, the first day of treatment for each group is designated as Study Day 0.

On the first day of each study, one mouse in Group 1 was treated with Trypsinogen/Chymotrypsinogen A at the lowest dose 2.6/15 mg/kg. As there were no prolonged severe adverse effects within two hours of treatment, the remaining three mice in this group were similarly treated.

As there were no prolonged severe adverse effects within 24 hours of initial treatment in Group 1 mice, one mouse in Group 2 was treated with Trypsinogen/Chymotrypsinogen A at 86.8/500 mg/kg for Study #2. As there were no visible signs of toxicity within two hours of this treatment, the remaining mice in each of these groups received the same treatment.

Treatment continued in this manner for the remaining dose of Trypsinogen/Chymotrypsinogen A for Study #2 (43.4/250 mg/kg) to animals in Group 3.

**Clinical Observations and Adverse Events for Study #2**

There were no adverse clinical signs observed in animals treated with Trypsinogen and Chymotrypsinogen A at 2.6/15, and 43.4/250 mg/kg; Groups 1 and 3, respectively) (Table 5).

Reddening of the skin on the tail and temporary minor clot formations in the tail vein at the injection site were the only adverse signs observed, occurring immediately following dosing in the highest dose group (Trypsinogen/Chymotrypsinogen A at 86.8/500 mg/kg) and resolved by the time of the next dose administration (Table 5). The clinical signs did not interfere with proper dose administration.

**Body Weight Changes**

There was mean body weight gain over the course of the study (Study Days 0 to 13) for each group (6.98%, 4.30% and 6.61% of initial weight for animals treated with Trypsinogen and Chymotrypsinogen A at 2.6/15, 86.8/500 and 43.4/250 mg/kg; Groups 1, 2 and 3, respectively) (Table 6 and Figure 2). No animals lost body weight in excess of 15% of initial weight (Table 7).

**Table 1: Dosing Regimen for Study #1**

| Group | Test Article | Treatment | Treatment Schedule |
|---|---|---|---|
| 1 | Trypsinogen/ Chymotrypsinogen A | 0.254/1.521 mg/kg in 10 mL/kg as a single i.v injection | Once daily for 7 days |
| 2 | Trypsinogen/ Chymotrypsinogen A | 0.381/2.282 mg/kg in 10 mL/kg as a single i.v. injection | Once daily for 7 days |
| 3 | Trypsinogen/ Chymotrypsinogen A | 0.572,3.42 mg/kg in 10 mL/kg as a single i.v. injection | Once daily for 7 days |

(continued)

| Group | Test Article | Treatment | Treatment Schedule |
|---|---|---|---|
| 4 | Trypsinogen/ Chymotrypsinogen A | 0.858/5.135 mg/kg in 10 mL/kg as a single i.v. injection | Once daily for 7 days |

Treatment was initiated to each group in a staggered manner, beginning with the lowest dose and then increasing the dose on each subsequent day. For all data calculations and presentation, the first day of treatment for each group is designated as Study Day 0.

**Table 2: Mean Body Weight Measurements ± SEM (g) for Each Treatment Group at Initiation and Termination of Study #1**

| Group | Treatment | Body Weight Study Day 0 (mean ± SEM) | Body Weight Final Day (mean ± SEM) | Delta Body Weight (%) | Survival Number |
|---|---|---|---|---|---|
| 1 | Trysinogen/ Chymotrypsinogen A 0.254/1.521 mg/kg | 19.8 ± 0.38 | 20.38 ± 0.43 | 2.59 | 4/4 |
| 2 | Trypsinogen/ Chymotrypsinogen A 0.381/2.282 mg/kg | 20.06±030 | 21.56 ± 0.30 | 7.48 | 4/4 |
| 3 | Trypsinogen/ Chymotrypsinogen A 0.572/3.423 mg/kg | 20.37 ± 0.11 | 21.99 0.31 | 7.97 | 4/4 |
| 4 | Trypsinogen/ Chymotrypsinogen A 0.859/5.135 mg/kg | 21.18 ± 0.40 | 21.61 ± 0.31 | 2.03 | 4/4 |

Treatments were administered as single i.v. injection, once daily for seven consecutive days. Treatment was initiated to each group in a staggered manner, beginning with the lowest dose and then increasing the dose on each subsequent day. For all data calculations and presentation, the first day of treatment for each group is designated as Study Day 0.

**Table 3: Initial Body Weight and Occurrence of Lowest Body Weight (g) for Each Animal During the Course of Study #1**

| Group | Treatment | Animal ID | Starting Weight (g) | Lowest Weight (g) | Days Post Initial Treatment | Delta Start to Lowest (%) |
|---|---|---|---|---|---|---|
| 1 | Trypsinogen/Chymotrypsinogen A 0.254/1.521 mg/kg | 07343 | 20.70 | 19.53 | 5 | -5.7 |
| | | 11414 | 19.87 | 19.70 | 12 | -0.9 |
| | | 37890 | 20.01 | 19.66 | 11 | -1.7 |
| | | 43929 | 18.87 | 18.87 | 0 | 0.0 |
| 2 | Trypsinogen/Chymotrypsinogen A 0381/2.282 mg/kg | 07510 | 19.70 | 19.62 | 4 | -0.4 |
| | | 15663 | 19.88 | 19.71 | 2 | -0.9 |
| | | 25443 | 20.95 | 20.95 | 0 | 0.0 |
| | | 45040 | 19.71 | 19.65 | 2 | -0.3 |
| 3 | Trypsinogen/Chymotrypsinogen A 0.572/3.423 mg/kg | 14447 | 20.48 | 20.48 | 0 | 0.0 |
| | | 17670 | 20.49 | 20.49 | 0 | 0.0 |
| | | 25190 | 20.46 | 20.46 | 0 | 0.0 |
| | | 45377 | 20.05 | 20.05 | 0 | 0.0 |

(continued)

| Group | Treatment | Animal ID | Starting Weight (g) | Lowest Weight (g) | Days Post Initial Treatment | Delta Start to Lowest (%) |
|---|---|---|---|---|---|---|
| 4 | Trypsinogen/Chymotrypsinogen A 0.858/5.135 mg/kg | 08584 | 21.25 | 21.25 | 0 | 0.0 |
| | | 20188 | 21.82 | 21.52 | 1 | -1.4 |
| | | 28900 | 20.02 | 19.19 | 2 | -4.1 |
| | | 31187 | 21.63 | 20.80 | 2 | -3.8 |

Treatments were administered as single i.v. injection, once daily for seven consecutive days.

Treatment was initiated to each group in a staggered manner, beginning with the lowest dose and then increasing the dose on each subsequent day. For all data calculations and presentation, the first day of treatment for each group is designated as Study Day 0.

**Table 4 : Dosing Regimen for Study #2**

| Group | Test Article | Treatment | Treatment Schedule |
|---|---|---|---|
| 1 | Trypsinogen/ Chymotrypsinogen A | 2.6/15 mg/kg in 10 mL/kg as a single i.v. injection | Once daily for 7 days |
| 2 | Trypsinogen/ Chymotrypsinogen A | 86.8/500 mg/kg in 10 mL/kg as a single i.v. injection | Once daily for 7 days |
| 3 | Trypsinogen/ Chymotiypsinogen A | 43.4/250 mg/kg in 10 mL/kg as a single i.v. injection | Once daily for 7 days |

Treatment was initiated to each group in a staggered manner, beginning with the lowest dose on the first day and then increasing the dose to Trypsinogen/Chymotrypsinogen A at 86.8/500 mg/kg on the second day. Treatment with Trypsinogen/Chyniotrypsinogen A at 43.4/250 mg/kg commenced on the third day. For all data calculations and presentation, the first day of treatment for each group is designated as Study Day 0.

**Table 5: Clinical Observations and Adverse Events for Study #2**

**Group 1: Trypsinogen/Chymotrypsinogen A 2.6/15 mg/kg**

| Animal ID | Observation Type | Description | Study Days Observed | Early termination |
|---|---|---|---|---|
| 3704333 | | | | |
| 3724622 | | | | |
| 3730389 | - | - | - | - |
| 3739400 | | | | |

**Group 2: Trypsinogen/Chymotypsinegen A at 86.8/5005 mg/kg**

| Animal ID | Observation Type | Description | Study Days Observed | Early termination |
|---|---|---|---|---|
| 3705776 | Skin and Fur | Reddened Skin on Tail | 2 to 4 | - |
| | Clotting appearing in dosed tail vein | - | 3 | |
| 3717536 | Skin and Fur | Reddened Skin on Tail | 1 to 5 | - |
| | Clotting appearing in dosed tail vein | - | 3 to 4 | |

(continued)

**Group 2: Trypsinogen/Chymotypsinegen A at 86.8/5005 mg/kg**

| | | | | |
|---|---|---|---|---|
| 3727819 | Skin and Fur | Reddened Skin on Tail | 1 to 5 | - |
| | Clotting appearing in dosed tail vein | - | 3 | |
| 3733560 | Skin and Fur | Reddened Skin on Tail | 1 to 5 | - |
| | Clotting appearing m dosed tail vein | - | 3 | |

**Group 3: Trypsinogen/Chymotrypsinogen A 43.4/250mg/kg**

| Animal ID | Observation Type | Description | Study Days Observed | Early termination |
|---|---|---|---|---|
| 3714640 3716014 3734449 3746801 | - | - | - | - |

Treatment was initiated to each group in a staggered manner, beginning with the lowest dose on the first day and then increasing the dose to Trypsinogen/Chymotrypsinogen A at 86.8/500 mg/kg on the second day. Treatment with Trypsinogen/Chymotrypsinogen A at 43.4/250 mg/kg commenced on the third day. For all data calculations and presentation, the first day of treatment for each group is designated as Study Day 0.

**Table 6: Mean Body Weight Measurements ± SEM (g) for Each Treatment Group at Initiation and Termination of Study #2**

| Group | Treatment | Body Weight Study Day 0 (mean ± SEM) | Body Weight Final Day (mean ± SEM) | Delta Body Weight (%) | Survival Number |
|---|---|---|---|---|---|
| 1 | Trypsinogen/ Chymotrypsinogen A 2.6/15mg/kg | 21.29 ± 0.89 | 22.77 ± 0.97 | 6.98 | 4/4 |
| 2 | Trypsinogen/ Chymotrypsinogen A 86.8/500 mg/kg | 21.23 ± 1.48 | 22.14 ± 1.52 | 4.30 | 4/4 |
| 3 | Trypsinogen/ Chymotrypsinogen A 43.4/250mg/kg | 20.70 ± 1.18 | 22.07 ± 0.95 | 6.61 | 4/4 |

Treatments were administered as single i.v. injection, once daily for seven consecutive days.
Treatment was initiated to each group in a staggered manner, beginning with the lowest dose on the first day and then increasing the dose to Trypsinogen/Chymotrypsinogen A at 86.8/500 mg/kg on the second day. Treatment with Trypsinogen/Chymotrypsinogen A at 43.4/250 mg/kg commenced on the third day. For all data calculations and presentation, the first day of treatment for each group is designated as Study Day 0.

**Table 7: Initial Body Weight and Occurrence of Lowest Body Weight (g) for Each Animal During the Course of Study #2**

| Group | Treatment | Animal ID | Starting Weight (g) | Lowest Weight (g) | Bays Post Initial Treatment | Delta Start to Lowest (%) |
|---|---|---|---|---|---|---|
| 1 | Tripsinogen/ Chymotrypsinogen A 2.6/15 mg/kg | 04333 | 20.89 | 20.21 | 1 | -3.3 |
| | | 24622 | 23.82 | 23.19 | 2 | -2.6 |
| | | 30389 | 19.64 | 19.40 | 2 | -1.2 |
| | | 39400 | 20.79 | 20.50 | 2 | -1.4 |
| 2 | Tlypsinogen/ Chymotrypsinogen A 86.8/500 mg/kg | 05176 | 18.85 | 18.73 | 1 | -0.6 |
| | | 17536 | 20.76 | 20.35 | 2 | -2.0 |
| | | 27819 | 25.50 | 24.83 | 2 and 7 | -2.6 |
| | | 33560 | 19.81 | 19.57 | 6 | -1.2 |
| 3 | Trypsinogen/ Chymotrypsinogen A 43.6/250 mg/kg | 14640 | 19.38 | 19.38 | 0 | 0.0 |
| | | 16014 | 20.08 | 20.09 | 0 | 0.0 |
| | | 34449 | 19.15 | 19.15 | 0 | 0.0 |
| | | 46801 | 14.20 | 24.18 | 5 | -0.1 |

Treatments were administered as single i.v. injection, once daily for seven consecutive days

Treatment was initiated to each group in a staggered manner, beginning with the lowest dose on the first day and then increasing the dose to Trypsinogen/Chymotrypsinogen A at 86.8/500 mg/kg on the second day Treatment with Trypsinogen/Chymotrypsinogen A at 43.4/250 mg/kg commenced on the third day. For all data calculations and presentation, the first day of treatment for each group is designated as Study Day 0.

**Example 3**

The study was performed to assess anti-tumour efficacy of Trypsinogen and Chymotrypsinogen A, administered in combination against Pan02 mouse pancreatic cancer cells, orthotopically inoculated in female C57BL/6 mice.

**Cell Line**

Pan02 mouse pancreatic tumour cells were sourced from National Cancer Institute (Frederick, MD, USA).

**Tumour Cell Culture**

Pan02 mouse pancreatic tumour cells (working stock VP-Stock 1235) were cultured in RPMI 1640 cell culture medium supplemented with 10% FBS, 1% GlutaMAX™ and 1% penicillin-streptomycin, and grown at 37°C in a humidified cell culture incubator supplied with 5% $CO_2$. The cells were harvested (Passage 7) by trypsinisation, washed twice in HBSS and counted (using trypan blue exclusion). The final cell density was adjusted with HBSS:Matrigel™ (1:1, v/v) to $5 \times 10^7$ Pan02 cells/mL.

The use of Matrigel™ in the inoculation suspension is known to support early vascularisation which can increase the take-rate of tumours, and therefore has the potential to decrease tumour size variability.

**Tumour** Cell Inoculation

Forty C57BL/6 female mice were inoculated while under intraperitoneally injected anaesthesia (Ketamine (14 mg/mL)/Xylazine (0.9 mg/mL)). Prior to inoculation, the skin at the incision site was swabbed with topical povidone iodine solution and then alcohol. An incision was made to expose the pancreas. A needle was introduced directly into the pancreas tail where 20 μL of cell suspension, consisting of $1 \times 10^6$ Pan02 cells, was discharged.

Mice were administered a 200 μL bolus dose of Buprenex (Buprenorphine HCl, 0.01 mg/mL) subcutaneously for pain relief at the time of surgery and the following day.

**Tumour Take-Rate Determination**

Ten randomly selected animals (Group 0) were euthanised seven days post inoculation to assess the size and take rate of tumours in the pancreas. The pancreas was excised and examined for the presence of a tumour.

The presence of a tumour was confirmed in the pancreas of all 10 of these animals.

**Randomisation**

Two days following confirmation of successful tumour take-rate, 30 of the remaining animals were randomised, based on body weight, into three groups of 10 (nine days post-inoculation; Study Day 0).

**Compound Formulation**

Trypsinogen and Chymotrypsinogen A were dissolved in phosphate buffered saline (PBS) on each treatment day to give stock solutions of 1 mg/mL. The dosing solutions of Trypsinogen/Chymotrypsinogen A at 2.75/16.5 and 8.33/50 mg/mL (ratio of 1:6) were prepared by diluting the Trypsinogen solution first in PBS and then adding Chymotrypsinogen A.

**Compound Administration**

The dosing regimen used in this study is summarised in Table 8.

The animals assigned for take-rate assessment (Group 0) remained untreated.

Vehicle Control (PBS; Groups 1) and Trypsinogen/Chymotrypsinogen A (in combination in a single injection) at doses of 27.5/165 (Group 2) and 83.3/500 mg/kg (Groups 3) were administered once daily i.v. in a dosing volume of 10 mL/kg. The volume of dosing solution administered to each animal was calculated and adjusted based on individual body weight measured immediately prior to dosing.

Treatments commenced on Study Day 0 (nine-days post-inoculation). Treatments were administered to animals in Groups 1, 2 and 3 for 26 consecutive days.

**Termination Procedure**

All take-rate assessment animals and those in Groups 1, 2 and 3 were euthanised via carbon dioxide inhalation by approved standard procedures.

**Sample Collection**

Upon termination, the pancreas was excised from all animals with tumour intact. The tumour was isolated from the pancreas tissue, weighed and then photographed (Figures 4 to 6). A 25 mg portion of tumour from each of the samples in Groups 4 and 5 were stored at -80°C and shipped to Adaptive Biotechnologies Corp. for further analysis.

**Calculations**

Mean percentage change in body weight (%BW Change) between Day 0 and any given day (Day X) was calculated using the equation:

$$\%BW\ Change = mean(BW_{Study\ Day\ X})/mean(BW_{Study\ Day\ 0}) \times 100\% - 100\%$$

$$where\ BW_{Study\ Day\ 0} = initial\ value\ on\ Day\ 0\ and\ BW_{Study\ Day\ X} = current\ value\ on\ Day\ X.$$

Percentage change in mean tumour weight for treated groups relative to the control group was calculated using the following equation:

$$Percentage\ change = ((Mean_{Control} - Mean_{Treatment})/Mean_{Control}) \times 100$$

**Statistical Calculations**

All statistical calculations were performed using Prism 6 for Mac OS X (GraphPad Software Inc, La Jolla, CA, USA).

Normality of all data-sets was tested using the Kolmogorov-Smirnov (KS) normality test.

A paired t-test was used to determine if body weight changed significantly within a treatment group between Study Day 0 and the termination day of each group.

Comparison of tumour weight at termination of the study was made between Groups 1, 2 and 3 using One-Way Analysis of Variance (ANOVA). Significant differences between groups were determined using Holm-Sidak's Multiple Comparisons Test.

A p value of $\leq 0.05$ was considered significant.

**Results and Observations**

**Body Weight Changes**

There was a small (not significant) mean body weight loss (0.54% of initial weight) in animals treated with Vehicle Control for 26 days (PBS; Group 1).

All other groups showed mean body weight gain; 0.82% and 2.72% of initial weight for groups treated with Trypsinogen/Chymotrypsinogen A at 27.5/165 and 83.3/500 mg/kg for 26 days (Groups 2 and 3, respectively).

**Efficacy of Compounds**

There was significant ($p\leq0.05$) reduction in mean tumour weight in animals treated for 26 days with Trypsinogen/Chymotrypsinogen A at 83.3/500 mg/kg (30.2 mg; 85.9% inhibition; Group 3) compared with Vehicle Control (PBS; 214.8 mg; Group 1), but not between Trypsinogen/Chymotrypsinogen A at 27.5/165 mg/kg (196.5 mg; 8.5 % inhibition; Group 2) and the Vehicle Control (Table 9 and Figure 3).

**Conclusion**

Anti-tumour efficacy of daily treatment with Trypsinogen and Chymotrypsinogen A, administered in combination as a single intravenous injection at doses of 27.5/165 or 83.3/500 mg/kg, was assessed against Pan02 mouse pancreatic cancer cells, orthotopically inoculated in female C57BL/6 mice.

Measurements of tumour weight at termination showed significant ($p\leq0.05$) anti-tumour efficacy in animals treated with high-dose Trypsinogen/Chymotrypsinogen A compared with control-treated animals after 26 days of treatment in this study.

**Table 8: Dosing Regimen**

| Group | Number of Animals | Test Article | Treatment | Treatment Schedule |
|---|---|---|---|---|
| 0 | 10 | No treatment (for take-rate assessment) | - | - |
| 1 | 10 | Vehicle Control (PBS) | 10 mL/kg, i.v. | Once daily (Study Days 0 to 25) |
| 2 | 10 | Trypsinogen/ Chymotrypsinogen A | 27.5/165 mg/kg in 10 mL/kg as a single i.v. injection | Once daily (Study Days 0 to 25) |
| 3 | 10 | Trypsinogen/ Chymotrypsinogen A | 83.3/500 mg/kg in 10 mL/kg as a single i.v. injection | Once daily (Study Days 0 to 25) |

**Table 9: Mean Tumour Weight ± SEM at Termination (mg) for Each Group**

| Group | Treatment | Termination Day | Number of Animals | Mean Tumour Weight ± SEM (mg) | Tumour inhibition* |
|-------|-----------|-----------------|-------------------|-------------------------------|--------------------|
| 1 | Vehicle Control (PBS) | 26 | 8 | 214.8 ± 23.4 | N/A |
| 2 | Trypsinogen/ Chymotrypsinogen A 21.5/165 mg/kg | 26 | 9 | 196.5 ± 30.5 | 8.5 |
| 3 | Trypsinogen/ Chymotrypsinogen A 83.3/500 mg/kg | 26 | 10 | 30.2 ± 8.2[a,b] | 85.9 |

Treatments were administered as a single i.v. injection, once daily (Study Days 0 to 25 for Groups 1, 2 and 3. Groups 1, 2 and 3 were terminated on Study Day 26. Tumours were not collected from animals that were found dead (one each in Groups 1 and 2). The value for one animal in Group 1 that was euthanised on Study Day 6 was not included in the analyses.

a p≤.05 compared with Group 1 (p<0.05; Holm-Sidak's Multiple Comparisons Test).

b p≤0.05 compared with Group 2 (p<0.05; Holm-Sidak's Multiple Comparisons Test).

Mean tumour weight ± SEM for each group are depicted in Figure 3.

* % Tumour inhibition was calculated for Groups 2 and 3 using Group 1 as the control.
N/A: not applicable

**Example 4**

The study was performed to assess anti-tumour efficacy of Trypsinogen and Chymotrypsinogen A, administered in combination, against A2780 human ovarian cancer cells orthotopically inoculated in female Athymic Nude-*Foxn1[nu]* mice.

**Cell Line**

A2780 human ovarian carcinoma cells were sourced from National Cancer Institute (Frederick, MD, USA).

**Tumour Cell Culture**

A2780 human ovarian cancer cells (working stock VP-Stock 1277) were cultured in DMEM supplemented with 10% FBS, 1% GlutaMAX™ and 1% penicillin-streptomycin, and grown at 37°C in a humidified cell culture incubator supplied with 5% $CO_2$. The cells were harvested (Passage 8) by trypsinisation, washed twice in HBSS and counted (using trypan blue exclusion). The final cell density was adjusted with HBSS:Matrigel™ (1:1, v/v) to $2.0 \times 10^8$ cells/mL.
The use of Matrigel™ in the inoculation suspension is known to support early vascularisation which can increase the take-rate of tumours, and therefore has the potential to decrease tumour size variability.

**Tumour Cell Inoculation**

Sixty athymic Nude-*Foxn1[nu]* mice were inoculated while under intraperitoneally injected anaesthesia (Ketamine (14 mg/mL)/Xylazine (0.9 mg/mL)). Prior to inoculation, the skin at the incision site was swabbed with alcohol. An incision was made to expose the ovary. A needle was introduced directly into the ovary where 5 μL of cell suspension, consisting of $1 \times 10^6$ A2780 cells was discharged.
Mice were administered a 200 μL bolus dose of Buprenex (Buprenorphine HCl, 0.01 mg/mL) subcutaneously for pain relief at the time of surgery and the following day.

**Randomisation**

The animals were randomised, based on body weight, into five groups of 12 and one group of 24, 7 days post-inoculation (Study Day 0).

## Compound Formulation

Trypsinogen and Chymotrypsinogen A were dissolved in phosphate buffered saline (PBS) on each treatment day to give stock solutions of 30 and 100 mg/mL, respectively. Dosing solution of Trypsinogen/Chymotrypsinogen A at 8.33/50, 2.75/16.5 and 0.91/5.4 mg/mL (ratio of 1:6) were prepared by diluting Trypsinogen solution first in PBS and then adding Chymotrypsinogen A.

## Compound Administration

The dosing regimen used in this study is summarised in Table 10.
Animals in Group 1 remained untreated for tumour take-rate assessment.
Vehicle Control (PBS; Group 2) and Trypsinogen and Chymotrypsinogen A at doses of 83.3/500, 27.5/165 and 9.1/54 mg/kg (Groups 3, 4 and 5, respectively; in combination in a single injection) were administered once daily via intravenous (i.v.) tail vein injection in a dosing volume of 10 mL/kg (12 animals per group). The volume of dosing solution administered to each animal was calculated and adjusted based on individual body weight measured immediately prior to dosing. Treatments commenced on Study Day 0 and were administered for 21 consecutive days.

## Tumour Take-Rate Determination

The untreated animals in Group 1 were euthanised seven days after the onset of treatment (14 days post-inoculation) to assess the size and take rate of tumours in the ovary.
The presence of a tumour was confirmed in the ovary of all 12 animals, then the tumour was isolated from the excised ovary and weighed for all animals. Tumour photographic images are presented in Figures 8 to 11.

## Termination Procedure

All animals were euthanised via carbon dioxide inhalation by approved standard procedures (Groups 1 through 5).

## Sample Collection

Upon termination, the ovary was excised from all animals with tumour intact. The tumour was isolated from the ovarian tissue and weighed. Tumours from animals receiving 21 days of treatment (Groups 2 through 5) were also photographed.

## Calculations

Mean percentage change in body weight (%BW Change) between Day 0 and any given day (Day X) was calculated using the equation:

$$\%BW\ Change = mean(BW_{Study\ Day\ X})/mean(BW_{Study\ Day\ 0}) \times 100\% - 100\%$$

$$where\ BW_{Study\ Day\ 0} = initial\ value\ on\ Day\ 0\ and\ BW_{Study\ Day\ X} = current\ value\ on\ Day\ X.$$

Percentage inhibition of tumour growth for treated groups relative to the control group was calculated using the following equation:

$$Percentage\ inhibition = ((Mean_{Control} - Mean_{Treatment})/Mean_{Control}) \times 100$$

## Statistical Calculations

All statistical calculations were performed using Prism 6 for Mac OS X (GraphPad Software Inc, La Jolla, CA, USA).
Normality of all data-sets was tested using the D'Agostino and Pearson omnibus normality test.

A paired t-test was used to determine if body weight changed significantly within groups receiving 21 days of treatment (Groups 2 through 5) between Study Day 0 and termination of the study. Where the data was not normally distributed, significance was determined using the Wilcoxon Matched Pairs Signed Rank Test.

Comparison of tumour weight at termination of the study was made between groups receiving 21 days of treatment (Groups 2 through 5) using One-Way Analysis of Variance (ANOVA).

A p value of $\leq 0.05$ was considered significant.

**Results and Observations**

**Body Weight Changes**

There was significant ($p \leq 0.05$) mean body weight gain between Study Days 0 and 7 in untreated animals (4.33% of initial weight; Group 1).

There was mean body weight gain between Study Days 0 and 21 in the groups treated for 21 days with Vehicle Control (PBS; 6.56% of initial weight; Group 2) and all doses of Trypsinogen/Chymotrypsinogen A (83.3/500, 27.5/165 and 9.1/54 mg/kg; 8.60%, 6.00% and 3.89% of initial weight; Groups 3, 4 and 5, respectively). Body weight gain was significant ($p \leq 0.05$) in all groups except low-dose treatment (Group 5).

**Efficacy of Compounds**

There was significant ($p \leq 0.05$) reduction in mean tumour weight in animals treated for 21 days with mid- and low-dose Trypsinogen/Chymotrypsinogen A (27.5/165 and 9.1/54 mg/kg; 957.3 and 1074.2 mg; 53.6% and 47.9%; Groups 4 and 5, respectively) compared with Vehicle Control (PBS; 2062.2 mg; Group 2), but not high-dose Trypsinogen/Chymotrypsinogen A (83.3/500 mg/kg; 1762.2 mg; 14.5%; Group 3) (Table 11 and Figure 7).

Tumour weights for untreated animals (Group 1) euthanised on Day 7 for take-rate assessment were not included in the analysis.

**Conclusion**

Anti-tumour efficacy of daily treatment with Trypsinogen and Chymotrypsinogen A, administered in combination as a single intravenous injection at doses of 83.3/500, 27.5/165 or 9.1/54 mg/kg, was assessed against A2780 human ovarian cancer cells, orthotopically inoculated in female athymic nude-*Foxn1$^{nu}$* mice.

Measurements of tumour weight at termination showed significant ($p \leq 0.05$) anti-tumour efficacy in animals treated with mid- and low-dose Trypsinogen/ Chymotrypsinogen A compared with Vehicle control-treated animals after 21 days of treatment in this study. Significant anti-tumour efficacy was not observed following high-dose treatment.

**Table 10: Dosing Regimen**

| Group | Test Article | Treatment | Treatment Schedule |
|---|---|---|---|
| 1 | No treatment (take-rate assessment) | - | - |
| 2 | Vehicle Control (PBS) | 10 mL/kg, i.v. | once daily (Study Days 0 to 20) |
| 3 | Trypsinogen/ Chymotrypsinogen A | 83.3/500 mg/kg in 10 mL/kg as a single i.v. injection | once daily (Study Days 0 to 20) |
| 4 | Trypsinogen/ Chymotrypsinogen A | 27.5/165 mg/kg in 10 mL/kg as a single i.v. injection | once daily (Study Days 0 to 20) |
| 5 | Trypsinogen/ Chymotrypsinogen A | 9/54 mg/kg in 10 mL/kg as a single i.v. injection | once daily (Study Days 0 to 20) |
| 6 | Trypsinogen/ Chymotrypsinogen A | 83.3/500 mg/kg in 10 mL/kg as a single i.v. injection | once on Study Day 19 |

**Table 11: Mean Tumour Weight ± SEM at Termination (mg) for Each Group**

| Group | Treatment | Number of Animals | Termination Day | Mean Tumour Weight ± SEM (mg) | % Tumour inhibition |
|---|---|---|---|---|---|
| 1 | No treatment (take-rate assessment) | 12 | 7 | 33.0±9.0 | N/A |
| 2 | Vehicle Control (PBS) | 12 | 21 | 2062.2 ± 331.4 | N/A |
| 3 | Tripsinogen/ Chymotrypsinogen A 83.3/500 mg/kg | 12 | 21 | 1762.2 ± 142.4 | 14.5 |
| 4 | Trypsinogen/ Chymotrypsinogen A 27.5/165 mg/kg | 12 | 21 | 957.3 ± 232.9a | 53.6 |
| 5 | Trypsinogen/ Chymotrypsinogen A 9.1/54 mg/kg | 12 | 21 | 1074.2 ± 225.5 [a] | 47.9 |

Untreated animals in Group 1 were euthanized on Study Day 7 (14 days post-inoculation) for tumour take-rate assessment. Tumour weights for animals in Group 1 were not included in the statistical analysis.

Treatments were administered to animals in Groups 2 through 5 as a single i.v. injection, once daily (Study Days 0 to 20). These animals were terminated on Study Day 21.

a: $p \leq 0.05$ compared with Group 2 (Holm-Sidak's Multiple Comparisons Test).

Mean tumour weight ± SEM for each group is depicted in Figure 7.

N/A: not applicable

## Example 5

Human dose conversion can be by any of the relevant methods described in U.S. Department of Health and Human Services Food and Drug Administration Center for Drug Evaluation and Research (CDER), Estimating the Maximum Safe Starting Dose in Initial Clinical Trials for Therapeutics in Adult Healthy Volunteers. Rockville, MD 2005. For example, the Human Equivalent Dose from a dose that has been administered to a mouse can be determined as follows:

Human Equivalent Dose (HED in mg/kg) = Animal Dose (mg/kg) × Animal K ÷ Human K, where K is a correction factor reflecting the relationship between body weight and body surface area.

For a typical adult (body weight 60 kg, body surface area $1.6m^2$), K is 37 and mouse K is 3. For example, 500mg/kg amount described herein can be converted to about 41 mg/kg for an equivalent human dose, and other exemplary conversions using this formula include:

Chymotrypsinogen:

Mouse (mg/kg): 1.5, 2, 3.5, 5, 15, 45, 135, 250 and 500.

Human (mg/kg): 0.12, 0.16, 0.28, 0.4, 1.2, 3.6, 11, 20 and 41

Trypsinogen:

Mouse (mg/kg): 0.25, 0.4, 0.6, 0.8, 2.5, 8, 20, 40 and 80.

Human (mg/kg): 0.02, 0.03, 0.05, 0.065, 0.2, 0.65, 1.6, 3.2 and 6.5.

Chymotrypsinogen:

Mouse (mg/kg): 15, 54 165, 250, 500.

Human (mg/kg): 1.2, 4.4, 13, 20 and 41.

Trypsinogen:

Mouse (mg/kg): 2.6, 9, 27.5, 43.4, 83.3, 86.8.

Human (mg/kg): 0.2, 0.7, 2.2, 3.5, 6.75 and 7.

The present invention includes human conversions for all amounts in mg/kg referred to herein, including Tables 1, 4, 8 and 10 and anywhere else in this document, based on human body weights of 50, 60, 70, 80, 90, 100 or more kg and body surface area of 1.1, 1.2, 1.3, 1.4, 1.5, 1.6, 1.7, 1.8 or more $m^2$.

The human equivalent doses described herein are derived using the methods described in the CDER 'Estimating the Maximum Safe Starting Dose in Initial Clinical Trials for Therapeutics in Adult Healthy Volunteers' for dose conversion based on body surface area. It will be appreciated that any variation on the methods described in the CDER document may also be utilised for determining an appropriate human dose from the dosages administered to mice, as described herein (for example, in some circumstances it may be appropriate to dose scaling based on body weight rather than on body surface area). Moreover, the skilled person will also be familiar with methods for determining the appropriate dose for a juvenile human (i.e., non-adult human), as well as methods for determining the appropriate dose in a non-human organism, to which the methods of the present invention may be applied. The skilled person will also be familiar with methods for adjusting the appropriate dose depending on the intended method of administration (for example, intravenous, intramuscular, subcutaneous, topical, oral or other method of administration).

## Example 6

The following experiments were performed to examine ratios of chymotrypsinogen to trypsinogen greater than 8:1.

## Materials and Methods

Materials

| Material | Supplier |
|---|---|
| Dulbecco's Modified Eagle Medium (DMEM), Eagle's Minimum Essential Medium (EMEM),Minimum Essential Medium (MEM), Roswell Park Memorial Institute (RPMI) 1640 cell culture medium, Foetal Bovine Serum (FBS), GlutaMAX™, sodium bicarbonate, penicillinstreptomycin (Pen/Strep) and trypsin | Invitrogen USA (Carlsbad, CA, USA) |
| CellTiter-Blue® Cell Viability Assay | Promega (Madison, WI, USA) |
| Trypan Blue | Sigma-Aldrich (St Louis, MO, USA) |

## Cell Culture

The following table (Table 12) shows the growth conditions and initial cell seeding densities in cells per well used in all ICso determination and combination assays. The cells were cultured at 37°C in a humidified cell culture incubator supplied with 95% air/5% $CO_2$.

| Cell line | Tumour Type | Cell culture medium | Seeding Density |
|---|---|---|---|
| 786-O | Kidney | RPMI+10% FBS+1% GlutaMAX™ +1% Pen/Strep | 400 |
| A2780| | Ovary | DMEM+10%FBS+1% GlutaMAX™ +1% Pen/Strep | 2500 |

(continued)

| Cell line | Tumour Type | Cell culture medium | Seeding Density |
|---|---|---|---|
| ACHN | Kidney | MEM+10%FBS+1% GlutaMAX-Fm+1% Pen/Strep | 4000 |
| BT-474 | Breast | RPMI+10% FBS+1% GlutaMAX™ +1% Pen/Strep +1% $NaHCO_3$ | 10000 |
| C8161.9 | Melanoma | DMEM+10% FBS+1% GlutaMAX™ +1% Pen/Strep | 1500 |
| DAOY | Brain | RPMI+10% FBS+1% GlutaMAX™ +1% Pen/Strep | 1500 |
| DU 145 | Prostate | RPMI+10% FBS+1% GlutaMAX™ +1% Pen/Strep | 800 |
| G-361 | Melanoma | RPMI+10% FBS+1% GlutaMAX™ +1% Pen/Strep | 3000 |
| HCT 116 | Colorectal | RPMI+10% FBS+1% GlutaMAX™ +1% Pen/Strep | 1500 |
| HCT-15 | Colorectal | RPMI+10% FBS+1% GlutaMAX™ +1% Pen/Strep | 1500 |
| Hep3B2.1-7 | Liver | RPMI+10% FBS+1% GlutaMAX™ +1% Pen/Strep | 1500 |
| HL-60 | Leukaemia | RPMI+10% FBS+1% GlutaMAX™ +1% Pen/Strep | 50000 |
| HT-1080 | Fibrosarcoma | EMEM+10% FBS+1% GlutaMAX™ +1% Pen/Strep | 1500 |
| HT-29 | Colorectal | RPMI+10% FBS+1% GlutaMAX™ +1% Pen/Strep | 4000 |
| HuH-7 | Liver | DMEM+10% FBS+1% GlutaMAX™ +1% Pen/Strep | 4000 |
| MCF-7 | Breast | RPMI+10% FBS+1% GlutaMAX™ +1% Pen/Strep | 5000 |
| MDA -MB-231 | Breast | DMEM+10% FBS+1% GlutaMAX™ +1% Pen/Strep | 6000 |
| MES-SA | Uterus | RPMI+10% FBS+1% GlutaMAX™ +1% Pen/Strep | 5000 |
| NCI-H460 | Lung | RPMI+10% FBS+1% GlutaMAX™ +1% Pen/Strep | 400 |
| NCI-H82 | Lung | RPMI+10% FBS+1% GlutaMAX™ +1% Pen/Strep | 25000 |
| PC-3 | Prostate | RPMI+10% FBS+1% GlutaMAX™ +1% Pen/Strep | 2500 |
| Raji | Leukaemia | RPMI+10% FBS+1% GlutaMAX™ +1% Pen/Strep | 3000 |
| SK-OV-3 | Ovary | RPMI+10% FBS+1% GlutaMAX™ +1% Pen/Strep | 1500 |
| SNB-19 | Brain | DMEM+10% FBS+1% GlutaMAX™ +1% Pen/Strep | 600 |
| U-87 MG | Brain | MEM+10% FBS+1% GlutaMAX™ +1% Pen/Strep | 1500 |

All following cell lines were sourced from American Type Culture Collection (ATCC) (Rockville, MD, USA): 786-0, ACHN, BT-474, DAOY, DU 145, G-361, HCT 116, HCT-15, Hep3B2.1-7, HL-60, HT-1080, HT-29, MCF-7, MDA-MB-231, MES-SA, NCI-H460, NCI-H82, PC-3, Raji, SK-OV-3, U-87 MG.

The A2780 cell line was sourced from the National Cancer Institute (NCI) (Bethesda, MD, USA).

The C8161.9 cell line was sourced from Dr. Gavin Robertson's Laboratory (College of Medicine, Pennsylvania State University, Hershey, PA, USA)).

The HuH-7 cell line was sourced from the Japanese Collection of Research Bioresources (JCRB) Cell Bank (Osaka, Japan).

The SNB-19 cell line was sourced from the Deutsche Sammlung von Mikroorganismen und Zellkulturen (DSMZ) (German Collection of Microorganisms and Cell Cultures) (Braunschweig, Germany).

All cell lines were utilized in assays up to passage 10.

**Test Articles**

**Test Article 1**

| Identity: | **Trypsinogen** |
|---|---|
| Description: | White powder |
| Lot Number: | OF001644 |
| Storage Conditions: | -20°C |
| Handling Precautions: | Standard laboratory precautions |
| Manufacturer / Supplier: | Applichem (Darmstadt, Germany) / Enzyme Supplies (Oxford, UK) |

**Test Article 2**

| Identity: | **Chymotrypsinogen** |
|---|---|
| Description: | White powder |
| Lot Number: | 3J006510 |
| Storage Conditions: | -20°C |
| Handling Precautions: | Standard laboratory precautions |
| Manufacturer / Supplier: | Applichem (Darmstadt, Germany) / Enzyme Supplies (Oxford, UK) |

**Test Article Formulation**

Trypsinogen and Chymotrypsinogen were dissolved directly in the appropriate cell culture medium and immediately added to the cells.

**Cell Growth Assays**

For combination assays, Test Articles were added to cells 24 hours post-seeding. Test Article concentrations were tested in triplicate for each cell line. Seventy-two hours post addition of Test Articles, the CellTiter-Blue® Assay was carried out on all plates.

The concentration of Trypsinogen used in the combination assays was based on a calculated $IC_{50}$ from single Test Article experiments. The concentration of Trypsinogen for each individual cell line determined the concentration of Chymotrypsinogen at the following ratios: 1:1, 1:2, 1:4, 1:6, 1:8 and 1:10 (Trypsinogen:Chymotrypsinogen). Controls consisted of growth medium only and untreated cells plus growth medium (untreated control).

Assay controls used were growth medium control as a vehicle and growth-medium only (background) as opposed to phosphate-buffered saline control as vehicle and Triton-x 100 as positive control. Background subtraction was not performed for the determination of $IC_{50}$ values.

Following incubation of cells in Test Article-containing media, 10 μL of CellTiter-Blue® was added to each well, then incubated with cells for up to 6 hours. Fluorescence was measured using a Spectramax Gemini XPS Fluorometer (560 nm excitation, 590 nm emission). All data were recorded and entered into Microsoft® Excel spreadsheets for interpretation.

**Calculations**

Data collected from CellTiter-Blue® assays were plotted as dose response curves for $IC_{50}$ determination. Relative Fluorescence Units (RFU) were plotted against compound concentrations. In these plots, the X-axis (compound concentration) was represented in a logarithmic scale. $IC_{50}$ concentration was calculated as the half maximal (50%) inhibitory concentration (IC) for each compound via a variable slope curve-fitting algorithm using GraphPad Prism version 6.0e for Mac OSX (GraphPad Software, San Diego California, USA).

For combination studies, the coefficient of drug interaction (CDI) was calculated according to the following equation:

$$CDI = \frac{TC}{T \, x \, C}$$

where TC is the growth inhibition of the combination of Trypsinogen and Chymotrypsinogen, T is the growth inhibition of the single agent Trypsinogen and C the growth inhibition of the single agent Chymotrypsinogen. CDI values below 1 indicate drug synergism, whereas values above 1 indicate an antagonistic interaction of Trypsinogen and Chymotrypsinogen.

**Cell Growth Inhibition by Trypsinogen and Chymotrypsinogen in Human Cancer**

The effect of Trypsinogen (T) and Chymotrypsinogen (C) alone or in combination on the growth of A2780 ovary tumour cells is shown in Figure 12. The greatest level of growth inhibition of A2780 ovary tumour cells was observed for ratios greater than 1:8 (T:C), for example 1:10 (T:C), see Table 13 (immediately below).

| A2780 | | Replicates (RFU) | | | Average | SEM | Growth Inhibition | CDI |
|---|---|---|---|---|---|---|---|---|
| | | 1 | 2 | 3 | | | | |
| | 1x Trypsinogen | 10871.031 | 10998.869 | 11247.885 | 11039.26 | 110.65 | 1.14 | |
| 1x Chymotrypsinogen | 9854.075 | 10037.875 | 9934.535 | 9942.16 | 53.20 | 1.03 | | |
| 2x Chymotrypsinogen | 7368.414 | 7412.912 | 8284.251 | 7688.53 | 298.14 | 0.80 | | |
| 4x Chymotrypsinogen | 5161.410 | 4959.459 | 5351.289 | 5157.39 | 113.13 | 0.53 | - | |
| 6x Chymotrypsinogen | 4068.919 | 3861.354 | 4293.539 | 4074.60 | 124.79 | 0.42 | | |
| 8x Chymotrypsinogen | 3145.061 | 2976.396 | 3189.652 | 3103.70 | 64.94 | 0.32 | | |
| 10x Chymotrypsinogen | 1531.170 | 1467.334 | 1503.991 | 1500.83 | 18.50 | 0.16 | | |
| 1:1 | 3569.706 | 3268.046 | 3522.157 | 3453.30 | 93.64 | 0.36 | 0.30 | |
| 1:2 | 1473.600 | 1497.48 | 1592.828 | 1521.30 | 36.42 | 0.16 | 0.17 | |
| 1:4 | 1060.256 | 1063.304 | 1112.314 | 1078.62 | 16.87 | 0.11 | 0.18 | |
| 1:6 | 1012.297 | 992.679 | 994.430 | 999.80 | 6.27 | 0.10 | 0.21 | |
| 1:8 | 959.808 | 879.302 | 939.566 | 926.23 | 24.18 | 0.10 | 0.26 | |
| 1:10 | 873.868 | 861.811 | 758.019 | 831.23 | 36.77 | 0.09 | 0.48 | |
| Control (cells only) | 9456.568 | 9771.397 | 9750.574 | 9659.51 | 101.65 | | | |
| Medium only | 721.110 | 709.561 | 680.464 | 703.71 | 12.09 | - | | |

The effect of Trypsinogen and Chymotrypsinogen alone or in combination on the growth of C8161.9 melanoma cells is shown in Figure 13. The greatest level of growth inhibition of C8161.9 melanoma cells was observed for ratios greater than 1:8 (T:C), for example 1:10 (T:C), see Table 14 (immediately below).

| C8161.9 | Replicates (RFU) | | | Average | SEM | Growth Inhibition | CDI |
|---|---|---|---|---|---|---|---|
| | 1 | 2 | 3 | | | | |
| 1x Trypsinogen | 8522.324 | 7631.593 | 8520.562 | 8224.83 | 296.62 | 1.10 | |
| 1x Chymotrypsinogen | 6777.939 | 8093.121 | 7808.435 | 7559.83 | 399.49 | 1.01 | |
| 2x Chymotrypsinogen | 6077.701 | 6742.977 | 6393.707 | 6404.80 | 192.13 | 0.85 | |
| 4x Chymotrypsinogen | 6515.904 | 6079.726 | 5879.160 | 6158.26 | 187.96 | 0.82 | - |
| 6x Chymotrypsinogen | 5416.613 | 5005.616 | 5229.323 | 5217.18 | 118.80 | 0.69 | |
| 8x Chymotrypsinogen | 7333.727 | 8090.684 | 8238.635 | 7887.68 | 280.25 | 1.05 | |
| 10x Chymotrypsinogen | 5673.008 | 6755.334 | 6653.124 | 6360.49 | 345.00 | 0.85 | |
| 1:1 | 5554.142 | 5775.944 | 6000.930 | 5777.01 | 128.98 | 0.77 | 0.70 |
| 1:2 | 3153.882 | 2757.958 | 3030.887 | 2980.91 | 116.99 | 0.40 | 0.42 |
| 1:4 | 1917.887 | 1922.872 | 2423.867 | 2088.21 | 167.84 | 0.28 | 0.31 |
| 1:6 | 1722.204 | 1741.576 | 1894.141 | 1785.97 | 54.37 | 0.24 | 0.31 |
| 1:8 | 1611.822 | 1638.031 | 1620.716 | 1623.52 | 7.69 | 0.22 | 0.19 |
| 1:10 | 1321.071 | 1166.744 | 1424.934 | 1304.25 | 75.01 | 0.17 | 0.19 |
| Control (cells only) | 7676.335 | 7617.46 | 7233.554 | 7509.12 | 138.83 | | |
| Medium only | 701.358 | 680.206 | 678.760 | 686.77 | 7.30 | - | |

The effect of Trypsinogen and Chymotrypsinogen alone or in combination on the growth of DAOY brain tumour cells shown in Figure 14. The greatest level of growth inhibition of DAOY brain tumour cells was observed for ratios greater than 1:8 (T:C), for example 1:10 (T:C), see Table 15 (immediately below).

| DAOY | Replicates (RFU) | | | Average | SEM | Growth Inhibition | CDI |
|---|---|---|---|---|---|---|---|
| | 1 | 2 | 3 | | | | |
| 1x Trypsinogen | 9051.549 | 9141.617 | 8347.920 | 8847.03 | 250.91 | 1.09 | |
| 1x Chymotrypsinogen | 8285.778 | 8192.939 | 8384.539 | 8287.75 | 55.32 | 1.02 | |
| 2x Chymotrypsinogen | 7865.063 | 7416.132 | 7612.021 | 7631.07 | 129.94 | 0.94 | |
| 4x Chymotrypsinogen | 5604.916 | 5300.233 | 5226.702 | 5377.28 | 115.78 | 0.66 | - |
| 6x Chymotrypsinogen | 4077.504 | 3677.683 | 3736.964 | 3830.72 | 124.57 | 0.47 | |
| 8x Chymotrypsinogen | 2896.918 | 2564.253 | 2455.450 | 2638.87 | 132.79 | 0.33 | |
| 10x Chymotrypsinogen | 1487.412 | 2023.845 | 2125.137 | 1878.80 | 197.87 | 0.23 | |
| 1:1 | 7861.021 | 7507.552 | 7543.847 | 7637.47 | 112.26 | 0.94 | 0.84 |
| 1:2 | 3425.338 | 3528.294 | 4562.312 | 3838.65 | 363.05 | 0.47 | 0.46 |
| 1:4 | 1142.231 | 1203.894 | 1154.619 | 1166.91 | 18.83 | 0.14 | 0.20 |
| 1:6 | 931.248 | 919.907 | 885.739 | 912.30 | 13.68 | 0.11 | 0.22 |
| 1:8 | 836.923 | 876.032 | 850.750 | 854.57 | 11.45 | 0.11 | 0.30 |
| 1:10 | 853.177 | 835.564 | 833.322 | 840.69 | 6.28 | 0.10 | 0.41 |
| Control (cells only) | 7932.601 | 8226.676 | 8164.348 | 8107.88 | 89.46 | | |
| Medium only | 771.175 | 766.154 | 457.132 | 664.82 | 103.85 | | - |

The effect of Trypsinogen and Chymotrypsinogen alone or in combination on the growth of DU145 prostate tumour cells shown in Figure 15. The greatest level of growth inhibition of DU145 prostate tumour cells was observed for ratios greater than 1:8 (T:C), for example 1:10 (T:C), see Table 16 (immediately below).

| DU145 | Replicates (RFU) | | | Average | SEM | Growth Inhibition | CDI |
|---|---|---|---|---|---|---|---|
| | 1 | 2 | 3 | | | | |
| 1x Trypsinogen | 12316.109 | 12798.373 | 11894.605 | 12336.36 | 261.09 | 1.04 | |
| 1x Chymotrypsinogen | 9641.914 | 9745.273 | 9408.376 | 9598.52 | 99.64 | 0.81 | |
| 2x Chymotrypsinogen | 6119.162 | 6511.184 | 6872.414 | 6500.92 | 217.51 | 0.55 | |
| 4x Chymotrypsinogen | 4067.276 | 4021.511 | 3791.056 | 3959.95 | 85.47 | 0.33 | - |
| 6x Chymotrypsinogen | 6162.657 | 6462.893 | 3916.473 | 5514.01 | 803.46 | 0.46 | |
| 8x Chymotrypsinogen | 6200.357 | 6068.926 | 6238.773 | 6169.35 | 51.42 | 0.52 | |
| 10x Chymotrypsinogen | 5037.879 | 4455.197 | 4268.117 | 4587.06 | 231.79 | 0.39 | |
| 1:1 | 8125.897 | 6246.690 | 8858.232 | 7743.61 | 777.74 | 0.65 | 0.78 |
| 1:2 | 3680.328 | 3374.181 | 3633.843 | 3562.78 | 95.25 | 0.30 | 0.53 |
| 1:4 | 2269.460 | 2366.647 | 2364.699 | 2333.60 | 32.08 | 0.20 | 0.57 |
| 1:6 | 1943.051 | 2036.505 | 1959.739 | 1979.77 | 28.78 | 0.17 | 0.35 |
| 1:8 | 1838.590 | 1705.612 | 1888.048 | 1810.75 | 54.47 | 0.15 | 0.28 |
| 1:10 | 1786.460 | 1756.369 | 1675.614 | 1739.48 | 33.09 | 0.15 | 0.36 |
| Control (cells only) | 12466.964 | 12147.758 | 10992.462 | 11869.06 | 447.88 | | |
| Medium only | 893.398 | 833.691 | 828.200 | 851.76 | 20.88 | - | |

The effect of Trypsinogen and Chymotrypsinogen alone or in combination on the growth of HCT 116 colorectal tumour cells shown in Figure 16. The greatest level of growth inhibition of HCT 116 colorectal tumour cells was observed for ratios greater than 1:8 (T:C), for example 1:10 (T:C), see Table 17 (immediately below).

| HCT 116 | Replicates (RFU) | | | Average | SEM | Growth Inhibition | CDI |
|---|---|---|---|---|---|---|---|
| | 1 | 2 | 3 | | | | |
| 1x Trypsinogen | 14859.604 | 15104.608 | 16112.500 | 15358.90 | 383.38 | 1.25 | |
| 1x Chymotrypsinogen | 12051.548 | 12853.539 | 13225.642 | 12710.24 | 346.42 | 1.04 | |
| 2x Chymotrypsinogen | 15683.443 | 16403.678 | 16092.573 | 16059.90 | 208.55 | 1.31 | |
| 4x Chymotrypsinogen | 11913.774 | 12127.43 | 11827.383 | 11956.20 | 89.18 | 0.97 | - |
| 6x Chymotrypsinogen | 8377.944 | 8716.581 | 9240.221 | 8778.25 | 250.82 | 0.71 | |
| 8x Chymotrypsinogen | 6522.517 | 6712.549 | 6865.853 | 6700.31 | 99.30 | 0.55 | |
| 10x Chymotrypsinogen | 4346.238 | 4803.888 | 4534.618 | 4561.58 | 132.80 | 0.37 | |
| 1:1 | 12396.237 | 12051.078 | 11672.182 | 12039.83 | 209.09 | 0.98 | 0.76 |
| 1:2 | 10120.919 | 10732.156 | 10003.201 | 10285.43 | 225.94 | 0.84 | 0.51 |
| 1:4 | 6582.828 | 6926.802 | 6857.768 | 6789.13 | 105.06 | 0.55 | 0.45 |
| 1:6 | 4158.295 | 3803.743 | 3997.355 | 3986.46 | 102.50 | 0.32 | 0.36 |
| 1:8 | 1306.618 | 1395.309 | 1404.950 | 1368.96 | 31.29 | 0.11 | 0.16 |
| 1:10 | 1173.282 | 1172.682 | 1199.737 | 1181.90 | 8.92 | 0.10 | 0.21 |
| Control (cells only) | 11788.172 | 12334.407 | 12717.700 | 12280.09 | 269.70 | | |
| Medium only | 853.945 | 862.254 | 852.246 | 856.15 | 3.09 | - | |

The effect of Trypsinogen and Chymotrypsinogen alone or in combination on the growth of Hep3B2.1-7 liver tumour cells shown in Figure 17. The greatest level of growth inhibition of Hep3B2.1-7 liver tumour cells was observed for ratios greater than 1:8 (T:C), for example 1:10 (T:C), see Table 18 (immediately below).

| Hep3B2.1-7 | Replicates (RFU) | | | Average | SEM | Growth Inhibition | CDI |
|---|---|---|---|---|---|---|---|
| | 1 | 2 | 3 | | | | |
| 1x Trypsinogen | 5763.579 | 5761.003 | 5278.852 | 5601.14 | 161.15 | 1.34 | |
| 1x Chymotrypsinogen | 3690.053 | 3975.591 | 3801.437 | 3822.36 | 83.09 | 0.92 | |
| 2x Chymotrypsinogen | 3829.325 | 3508.687 | 3595.125 | 3644.38 | 95.78 | 0.87 | |

(continued)

| Hep3B2.1-7 | Replicates (RFU) | | | Average | SEM | Growth Inhibition | CDI |
|---|---|---|---|---|---|---|---|
| | 1 | 2 | 3 | | | | |
| 4x Chymotrypsinogen | 3349.295 | 3516.303 | 3482.731 | 3449.44 | 51.00 | 0.83 | - |
| 6x Chymotrypsinogen | 3271.956 | 3098.025 | 3087.413 | 3152.46 | 59.82 | 0.75 | |
| 8x Chymotrypsinogen | 2348.604 | 2595.240 | 2850.457 | 2598.10 | 144.88 | 0.62 | |
| 10x Chymotrypsinogen | 2513.241 | 2617.293 | 2788.512 | 2639.68 | 80.25 | 0.63 | |
| 1:1 | 3980.236 | 4263.435 | 3773.279 | 4005.65 | 142.07 | 0.96 | 0.78 |
| 1:2 | 3308.778 | 2702.337 | 3030.569 | 3013.89 | 175.26 | 0.72 | 0.62 |
| 1:4 | 1182.575 | 1158.461 | 1209.168 | 1183.40 | 14.64 | 0.28 | 0.26 |
| 1:6 | 937.534 | 893.343 | 919.586 | 916.82 | 12.83 | 0.22 | 0.22 |
| 1:8 | 863.658 | 868.435 | 894.195 | 875.43 | 9.48 | 0.21 | 0.25 |
| 1:10 | 862.721 | 844.240 | 857.605 | 854.86 | 5.51 | 0.20 | 0.24 |
| Control (cells only) | 4078.486 | 4214.781 | 4236.280 | 4176.52 | 49.41 | | |
| Medium only | 695.284 | 697.555 | 695.303 | 696.05 | 0.75 | | - |

The effect of Trypsinogen and Chymotrypsinogen alone or in combination on the growth of HT-29 colorectal tumour cells shown in Figure 18. The greatest level of growth inhibition of HT-29 colorectal tumour cells was observed for ratios greater than 1:8 (T:C), for example 1:10 (T:C), see Table 19 (immediately below).

| HT-29 | Replicates (RFU) | | | Average | SEM | Growth Inhibition | CDI |
|---|---|---|---|---|---|---|---|
| | 1 | 2 | 3 | | | | |
| 1x Trypsinogen | 8223.840 | 8376.067 | 8518.233 | 8372.71 | 85.00 | 0.86 | |
| 1x Chymotrypsinogen | 4538.796 | 4481.600 | 4338.855 | 4453.08 | 59.45 | 0.46 | |
| 2x Chymotrypsinogen | 13525.958 | 14096.041 | 13637.257 | 13753.09 | 174.46 | 1.42 | |
| 4x Chymotrypsinogen | 10580.120 | 10971.600 | 10771.067 | 10774.26 | 113.02 | 1.11 | - |
| 6x Chymotrypsinogen | 8547.735 | 7930.625 | 8223.705 | 8234.02 | 178.22 | 0.85 | |
| 8x Chymotrypsinogen | 5538.742 | 6045.101 | 5833.865 | 5805.90 | 146.84 | 0.60 | |
| 10x Chymotrypsinogen | 4538.783 | 4299.262 | 4388.604 | 4408.88 | 69.88 | 0.45 | |
| 1:1 | 9352.964 | 9423.387 | 9389.121 | 9388.49 | 20.33 | 0.97 | 2.44 |
| 1:2 | 7848.395 | 7681.472 | 8119.716 | 7883.19 | 127.70 | 0.81 | 0.66 |
| 1:4 | 5051.926 | 5072.896 | 5130.757 | 5085.19 | 23.57 | 0.52 | 0.55 |
| 1:6 | 3426.633 | 3235.928 | 3040.573 | 3234.38 | 111.45 | 0.33 | 0.46 |
| 1:8 | 3505.802 | 3421.350 | 3548.355 | 3491.84 | 37.32 | 0.36 | 0.70 |
| 1:10 | 3010.496 | 3166.377 | 3032.058 | 3069.64 | 48.77 | 0.32 | 0.81 |
| Control (cells only) | 8815.367 | 9695.704 | 10592.513 | 9701.19 | 513.03 | | |
| Medium only | 812.770 | 807.322 | 813.374 | 811.16 | 1.92 | | - |

The effect of Trypsinogen and Chymotrypsinogen alone or in combination on the growth of HuH-7 liver tumour cells shown in Figure 19. The greatest level of growth inhibition of HuH-7 liver tumour cells was observed for ratios greater than 1:8 (T:C), for example 1:10 (T:C), see Table 20 (immediately below).

| HuH-7 | Replicates (RFU) | | | Average | SEM | Growth Inhibition | CDI |
|---|---|---|---|---|---|---|---|
| | 1 | 2 | 3 | | | | |
| 1x Trypsinogen | 11847.077 | 11647.785 | 11902.568 | 11799.14 | 77.36 | 1.14 | |
| 1x Chymotrypsinogen | 9654.204 | 9996.031 | 10270.954 | 9973.73 | 178.39 | 0.97 | |
| 2x Chymotrypsinogen | 8675.452 | 8331.810 | 9428.336 | 8811.87 | 323.80 | 0.85 | |
| 4x Chymotrypsinogen | 7352.330 | 6400.635 | 7106.839 | 6953.27 | 285.26 | 0.67 | - |
| 6x Chymotrypsinogen | 6385.941 | 6303.662 | 6554.035 | 6414.55 | 73.68 | 0.62 | |

(continued)

| HuH-7 | Replicates (RFU) | | | Average | SEM | Growth Inhibition | CDI |
|---|---|---|---|---|---|---|---|
| | **1** | **2** | **3** | | | | |
| 8x Chymotrypsinogen | 7693.982 | 7100.407 | 7891.567 | 7561.99 | 237.73 | 0.73 | |
| 10x Chymotrypsinogen | 3056.197 | 3186.256 | 3368.272 | 3203.58 | 90.50 | 0.31 | |
| 1:1 | 3151.837 | 2987.080 | 3515.283 | 3218.07 | 156.03 | 0.31 | 0.28 |
| 1:2 | 1513.124 | 1461.279 | 1502.350 | 1492.25 | 15.80 | 0.14 | 0.15 |
| 1:4 | 1020.535 | 1034.911 | 1061.719 | 1039.06 | 12.07 | 0.10 | 0.13 |
| 1:6 | 950.172 | 923.260 | 928.811 | 934.08 | 8.20 | 0.09 | 0.13 |
| 1:8 | 962.377 | 980.744 | 969.830 | 970.98 | 5.33 | 0.09 | 0.11 |
| 1:10 | 943.712 | 902.317 | 924.857 | 923.63 | 11.97 | 0.09 | 0.25 |
| Control (cells only) | 10366.099 | 10304.473 | 10314.27 | 10328.28 | 19.12 | | |
| Medium only | 783.754 | 745.053 | 749.117 | 759.31 | 12.28 | - | |

The effect of Trypsinogen and Chymotrypsinogen alone or in combination on the growth of NCI-H460 lung tumour cells shown in Figure 20. The greatest level of growth inhibition of NCI-H460 lung tumour cells was observed for ratios greater than 1:8 (T:C), for example 1:10 (T:C), see Table 21 (immediately below).

| NCI-H460 | Replicates (RFU) | | | Average | SEM | Growth Inhibition | CDI |
|---|---|---|---|---|---|---|---|
| | **1** | **2** | **3** | | | | |
| 1x Trypsinogen | 12766.405 | 13907.190 | 13715.400 | 13463.00 | 352.67 | 1.08 | |
| 1x Chymotrypsinogen | 9938.641 | 11144.394 | 11179.673 | 10754.24 | 407.92 | 0.87 | |
| 2x Chymotrypsinogen | 7917.386 | 6785.913 | 7954.079 | 7552.46 | 383.42 | 0.61 | |
| 4x Chymotrypsinogen | 6269.978 | 6384.694 | 6637.457 | 6430.71 | 108.55 | 0.52 | - |
| 6x Chymotrypsinogen | 6382.878 | 5722.162 | 6237.417 | 6114.15 | 200.44 | 0.49 | |
| 8x Chymotrypsinogen | 7626.570 | 8267.497 | 7096.047 | 7663.37 | 338.67 | 0.62 | |
| 10x Chymotrypsinogen | 6828.383 | 5910.312 | 6399.807 | 6379.50 | 265.22 | 0.51 | |
| 1:1 | 7832.132 | 6908.952 | 8165.215 | 7635.43 | 375.75 | 0.61 | 0.66 |
| 1:2 | 4780.923 | 5036.047 | 4030.338 | 4615.77 | 301.84 | 0.37 | 0.56 |
| 1:4 | 3010.435 | 2684.551 | 2205.659 | 2633.55 | 233.71 | 0.21 | 0.38 |
| 1:6 | 1976.275 | - | 1786.417 | 1285.22 | 94.93 | 0.15 | 0.28 |
| 1:8 | 1589.983 | 1128.840 | 1940.589 | 1553.14 | 235.05 | 0.12 | 0.19 |
| 1:10 | 1021.508 | 1142.525 | 954.422 | 1039.49 | 55.04 | 0.08 | 0.15 |
| Control (cells only) | 12357.635 | 12437.118 | 12494.679 | 12429.81 | 39.73 | | |
| Medium only | 859.583 | 856.347 | 840.046 | 851.99 | 6.05 | - | |

## Claims

1. A pharmaceutical composition for use in the treatment of cancer, the composition comprising chymotrypsinogen, trypsinogen and a pharmaceutically acceptable diluent, excipient or carrier, wherein the chymotrypsinogen is administered in an amount of equal to, or greater than, 3.5 mg/kg, and no more than 41 mg/kg and the trypsinogen is administered in an amount of equal to, or greater than, 0.6 mg/kg and no more than 7 mg/kg.

2. The pharmaceutical composition for the use of claim 1, wherein the cancer is any one of pancreatic cancer, oesophageal cancer, colon cancer, bowel cancer, prostate cancer, ovarian cancer, stomach cancer, breast cancer, liver cancer, malignant melanoma, fibrosarcoma or lung cancer, preferably wherein the cancer is pancreatic cancer or ovarian cancer.

3. The pharmaceutical composition for the use of claim 1 or 2, wherein the amount of chymotrypsinogen is greater than 10 mg/kg, 20 mg/kg or 40 mg/kg;

and/or

wherein the amount of trypsinogen is greater than 1.5 mg/kg, 3 mg/kg, or 6 mg/kg.

4. The pharmaceutical composition for the use of claim 3, wherein the amount of chymotrypsinogen is equal to or greater than 13 mg/kg, and the amount of trypsinogen is equal to or amount greater than 2.2 mg/kg.

5. The pharmaceutical composition for the use of claim 3, wherein the amount of chymotrypsinogen is 41 mg/kg and the amount trypsinogen is 6.75 mg/kg, for example, wherein chymotrypsinogen is an amount of about 41 mg/kg and trypsinogen is an amount of about 7 mg/kg.

6. A pharmaceutical composition for use in the treatment of pancreatic cancer, the composition comprising chymotrypsinogen, trypsinogen and a pharmaceutically acceptable diluent, excipient or carrier, wherein the composition is adapted to administer chymotrypsinogen in an amount of equal to, or greater than 5mg/kg, and no more than 41 mg/kg and trypsinogen in an amount of equal to, or greater than, 1.5mg/kg and no more than 7 mg/kg.

7. The pharmaceutical composition for the use of claim 6, wherein chymotrypsinogen is an amount of greater than 13mg/kg, and trypsinogen in an amount greater than 2 mg/kg.

8. The pharmaceutical composition for the use of claim 6 or 7, wherein chymotrypsinogen is administered in an amount of about 41 mg/kg and trypsinogen is administered in an amount of about 7 mg/kg.

9. A pharmaceutical composition for use in the treatment of ovarian cancer, the composition comprising chymotrypsinogen, trypsinogen and a pharmaceutically acceptable diluent, excipient or carrier, wherein the composition is adapted to administer chymotrypsinogen in an amount of equal to, or greater than, 4mg/kg and no more than 41 mg/kg and trypsinogen in an amount of equal to, or greater than, 0.7 mg/kg, and no more than 7 mg/kg.

10. The pharmaceutical composition for the use of claim 9, wherein chymotrypsinogen is an amount of no more than 13 mg/kg and trypsinogen is an amount of no more than 2.5 mg/kg, for example, wherein chymotrypsinogen is an amount of 13 mg/kg and trypsinogen is an amount of 2.2 mg/kg.

11. The pharmaceutical composition for the use of any one of claims 1 to 10, wherein chymotrypsinogen and trypsinogen is administered in a weight ratio in the range of between about 4 : 1 to 8 : 1.

12. The pharmaceutical composition for the use of any one of claims 1 to 10, wherein chymotrypsinogen and trypsinogen is administered in a weight ratio between about 5 : 1 to 7 : 1.

13. The pharmaceutical composition for the use of any one of claims 1 to 10, wherein chymotrypsinogen and trypsinogen is administered in a weight ratio of about 6 : 1.

14. The pharmaceutical composition for the use of any one of claims 1 to 10, wherein chymotrypsinogen and trypsinogen is administered in a weight ratio greater than 8 : 1.

15. The pharmaceutical composition for the use of claim 14, wherein the weight ratio is greater than 10:1.

16. The pharmaceutical composition for the use of claim 14, wherein the weight ratio is 10 : 1.

17. The pharmaceutical composition for the use of any one of claims 1 to 16 wherein the composition does not contain amylase.

**Patentansprüche**

1. Pharmazeutische Zusammensetzung zur Verwendung bei der Behandlung von Krebs, wobei die Zusammensetzung Chymotrypsinogen, Trypsinogen und ein pharmazeutisch annehmbares Verdünnungsmittel, Hilfsstoff oder Träger umfasst, wobei das Chymotrypsinogen in einer Menge von gleich oder größer als 3,5 mg/kg und nicht mehr als 41 mg/kg verabreicht wird und das Trypsinogen in einer Menge von gleich oder größer als 0,6 mg/kg und nicht mehr als 7 mg/kg verabreicht wird.

**2.** Pharmazeutische Zusammensetzung zur Verwendung nach Anspruch 1, wobei der Krebs einer der folgenden ist: Bauchspeicheldrüsenkrebs, Speiseröhrenkrebs, Dickdarmkrebs, Darmkrebs, Prostatakrebs, Eierstockkrebs, Magenkrebs, Brustkrebs, Leberkrebs, malignes Melanom, Fibrosarkom oder Lungenkrebs, wobei der Krebs vorzugsweise Bauchspeicheldrüsenkrebs oder Eierstockkrebs ist.

**3.** Pharmazeutische Zusammensetzung zur Verwendung nach Anspruch 1 oder 2,

wobei die Menge an Chymotrypsinogen größer als 10 mg/kg, 20 mg/kg oder 40 mg/kg ist; und/oder
wobei die Menge an Trypsinogen größer als 1,5 mg/kg, 3 mg/kg oder 6 mg/kg ist.

**4.** Pharmazeutische Zusammensetzung zur Verwendung nach Anspruch 3, wobei die Menge an Chymotrypsinogen gleich oder größer als 13 mg/kg ist und die Menge an Trypsinogen gleich oder größer als 2,2 mg/kg ist.

**5.** Pharmazeutische Zusammensetzung zur Verwendung nach Anspruch 3, wobei die Menge an Chymotrypsinogen 41 mg/kg beträgt und die Menge an Trypsinogen 6,75 mg/kg beträgt, wobei beispielsweise Chymotrypsinogen in einer Menge von etwa 41 mg/kg und Trypsinogen in einer Menge von etwa 7 mg/kg vorliegt.

**6.** Pharmazeutische Zusammensetzung zur Verwendung bei der Behandlung von Bauchspeicheldrüsenkrebs, wobei die Zusammensetzung Chymotrypsinogen, Trypsinogen und ein pharmazeutisch annehmbares Verdünnungsmittel, Hilfsstoff oder Träger umfasst, wobei die Zusammensetzung angepasst ist, um Chymotrypsinogen in einer Menge von gleich oder größer als 5 mg/kg und nicht mehr als 41 mg/kg und Trypsinogen in einer Menge von gleich oder größer als 1,5 mg/kg und nicht mehr als 7 mg/kg zu verabreichen.

**7.** Pharmazeutische Zusammensetzung zur Verwendung nach Anspruch 6, wobei Chymotrypsinogen in einer Menge von mehr als 13 mg/kg und Trypsinogen in einer Menge von mehr als 2 mg/kg vorliegt.

**8.** Pharmazeutische Zusammensetzung zur Verwendung nach Anspruch 6 oder 7, wobei Chymotrypsinogen in einer Menge von etwa 41 mg/kg verabreicht wird und Trypsinogen in einer Menge von etwa 7 mg/kg verabreicht wird.

**9.** Pharmazeutische Zusammensetzung zur Verwendung bei der Behandlung von Eierstockkrebs, wobei die Zusammensetzung Chymotrypsinogen, Trypsinogen und ein pharmazeutisch annehmbares Verdünnungsmittel, Hilfsstoff oder Träger umfasst, wobei die Zusammensetzung angepasst ist, um Chymotrypsinogen in einer Menge von gleich oder größer als 4 mg/kg und nicht mehr als 41 mg/kg und Trypsinogen in einer Menge von gleich oder größer als 0,7 mg/kg und nicht mehr als 7 mg/kg zu verabreichen.

**10.** Pharmazeutische Zusammensetzung zur Verwendung nach Anspruch 9, wobei Chymotrypsinogen in einer Menge von nicht mehr als 13 mg/kg und Trypsinogen in einer Menge von nicht mehr als 2,5 mg/kg vorliegt, wobei beispielsweise Chymotrypsinogen in einer Menge von 13 mg/kg und Trypsinogen in einer Menge von 2,2 mg/kg vorliegt.

**11.** Pharmazeutische Zusammensetzung zur Verwendung nach einem der Ansprüche 1 bis 10, wobei Chymotrypsinogen und Trypsinogen in einem Gewichtsverhältnis im Bereich von etwa 4 : 1 bis 8 : 1 verabreicht werden.

**12.** Pharmazeutische Zusammensetzung zur Verwendung nach einem der Ansprüche 1 bis 10, wobei Chymotrypsinogen und Trypsinogen in einem Gewichtsverhältnis von etwa 5 : 1 bis 7 : 1 verabreicht werden.

**13.** Pharmazeutische Zusammensetzung zur Verwendung nach einem der Ansprüche 1 bis 10, wobei Chymotrypsinogen und Trypsinogen in einem Gewichtsverhältnis von etwa 6 : 1 verabreicht werden.

**14.** Pharmazeutische Zusammensetzung zur Verwendung nach einem der Ansprüche 1 bis 10, wobei Chymotrypsinogen und Trypsinogen in einem Gewichtsverhältnis von mehr als 8 : 1 verabreicht werden.

**15.** Pharmazeutische Zusammensetzung zur Verwendung nach Anspruch 14, wobei das Gewichtsverhältnis größer als 10 : 1 ist.

**16.** Pharmazeutische Zusammensetzung zur Verwendung nach Anspruch 14, wobei das Gewichtsverhältnis 10 : 1 beträgt.

**17.** Pharmazeutische Zusammensetzung zur Verwendung nach einem der Ansprüche 1 bis 16, wobei die Zusammensetzung keine Amylase enthält.

**Revendications**

1. Composition pharmaceutique destinée à être utilisée dans le traitement du cancer, la composition comprenant du chymotrypsinogène, du trypsinogène et un diluant, excipient ou véhicule pharmaceutiquement acceptable, dans laquelle le chymotrypsinogène est administré en une quantité égale ou supérieure à 3,5 mg/kg, et pas supérieure à 41 mg/kg et le trypsinogène est administré en une quantité égale ou supérieure à 0,6 mg/kg et pas supérieure à 7 mg/kg.

2. Composition pharmaceutique destinée à être utilisée selon la revendication 1, dans laquelle le cancer est l'un quelconque du cancer du pancréas, du cancer de l'œsophage, du cancer du côlon, du cancer de l'intestin, du cancer de la prostate, du cancer de l'ovaire, du cancer de l'estomac, du cancer du sein, du cancer du foie, du mélanome malin, du fibrosarcome ou du cancer du poumon, de préférence dans laquelle le cancer est le cancer du pancréas ou le cancer de l'ovaire.

3. Composition pharmaceutique destinée à être utilisée selon la revendication 1 ou 2, dans laquelle la quantité de chymotrypsinogène est supérieure à 10 mg/kg, 20 mg/kg ou 40 mg/kg;
et/ou
dans laquelle la quantité de trypsinogène est supérieure à 1,5 mg/kg, 3 mg/kg ou 6 mg/kg.

4. Composition pharmaceutique destinée à être utilisée selon la revendication 3, dans laquelle la quantité de chymotrypsinogène est égale ou supérieure à 13 mg/kg, et la quantité de trypsinogène est égale ou supérieure à 2,2 mg/kg.

5. Composition pharmaceutique destinée à être utilisée selon la revendication 3, dans laquelle la quantité de chymotrypsinogène est 41 mg/kg et la quantité de trypsinogène est 6,75 mg/kg, par exemple, dans laquelle le chymotrypsinogène est une quantité d'environ 41 mg/kg et le trypsinogène est une quantité d'environ 7 mg/kg.

6. Composition pharmaceutique destinée à être utilisée dans le traitement du cancer du pancréas, la composition comprenant du chymotrypsinogène, du trypsinogène et un diluant, excipient ou véhicule pharmaceutiquement acceptable, dans laquelle la composition est adaptée pour administrer le chymotrypsinogène en une quantité égale ou supérieure à 5 mg/kg et pas supérieure à 41 mg/kg et le trypsinogène en une quantité égale ou supérieure à 1,5 mg/kg et pas supérieure à 7 mg/kg.

7. Composition pharmaceutique destinée à être utilisée selon la revendication 6, dans laquelle le chymotrypsinogène est une quantité supérieure à 13 mg/kg, et le trypsinogène en une quantité supérieure à 2 mg/kg.

8. Composition pharmaceutique destinée à être utilisée selon la revendication 6 ou 7, dans laquelle le chymotrypsinogène est administré en une quantité d'environ 41 mg/kg et le trypsinogène est administré en une quantité d'environ 7 mg/kg.

9. Composition pharmaceutique destinée à être utilisée dans le traitement du cancer de l'ovaire, la composition comprenant du chymotrypsinogène, du trypsinogène et un diluant, excipient ou véhicule pharmaceutiquement acceptable, dans laquelle la composition est adaptée pour administrer le chymotrypsinogène en une quantité égale ou supérieure à 4 mg/kg et pas supérieure à 41 mg/kg et le trypsinogène en une quantité égale ou supérieure à 0,7 mg/kg et pas supérieure à 7 mg/kg.

10. Composition pharmaceutique destinée à être utilisée selon la revendication 9, dans laquelle le chymotrypsinogène est une quantité pas supérieure à 13 mg/kg et le trypsinogène est une quantité pas supérieure à 2,5 mg/kg, par exemple, dans laquelle le chymotrypsinogène est une quantité de 13 mg/kg et le trypsinogène est une quantité de 2,2 mg/kg.

11. Composition pharmaceutique destinée à être utilisée selon l'une quelconque des revendications 1 à 10, dans laquelle le chymotrypsinogène et le trypsinogène sont administrés dans un rapport pondéral dans la plage entre environ 4 : 1 et 8 : 1.

**12.** Composition pharmaceutique destinée à être utilisée selon l'une quelconque des revendications 1 à 10, dans laquelle le chymotrypsinogène et le trypsinogène sont administrés dans un rapport pondéral entre environ 5 : 1 et 7 : 1.

**13.** Composition pharmaceutique destinée à être utilisée selon l'une quelconque des revendications 1 à 10, dans laquelle le chymotrypsinogène et le trypsinogène sont administrés dans un rapport pondéral d'environ 6 : 1.

**14.** Composition pharmaceutique destinée à être utilisée selon l'une quelconque des revendications 1 à 10, dans laquelle le chymotrypsinogène et le trypsinogène sont administrés dans un rapport pondéral supérieur à 8 : 1.

**15.** Composition pharmaceutique destinée à être utilisée selon la revendication 14, dans laquelle le rapport pondéral est supérieur à 10 : 1.

**16.** Composition pharmaceutique destinée à être utilisée selon la revendication 14, dans laquelle le rapport pondéral est 10 : 1.

**17.** Composition pharmaceutique destinée à être utilisée selon l'une quelconque des revendications 1 à 16 dans laquelle la composition ne contient pas d'amylase.

## Figure 1: Mean Body Weight ± SEM (g) for Each Group During Study #1

**Figure 2: Mean Body Weight ± SEM (g) for Each Group During Study #2**

**Figure 3:**

**Figure 4:**

Group 1 (PBS, 10 ml/kg, daily i.v) Day 26

5867521  5874497  5873973  5878707

5883631  5891232  5891281  5895606

1 cm

**Figure 5:**

Group 2 (T:C, 27.5 mg/kg:165 mg/kg) Day 26

5862359          5865983          5872646          5875516

5878042          5885211          5888253          5890091

5897691

1 cm

**Figure 6:**

## Group 3 (T:C, 83.3 mg/kg:500 mg/kg) Day 26

5865644     5866535     5875123     5878925

5884566     5891204     5894394     5898933

5900402     5902681

1 cm

**Figure 7**

**Figure 8**

3710961  3712594  3713507  3718011  3722791

3723439  3724287  3726283  3733385  3735781

3739074  3742064

10 mm

**Figure 9**

3710299  3716411  3718487  3718829  3724380

3725382  3728913  3731980  3732762  3736767

3741335  3743600

10 mm

**Figure 10**

3711361      3712207      3719750      3722441      3728856

3730134      3731281      3732143      3734230      3736791

10 mm

**Figure 11**

3707810      3711377      3711536      3712927      3729006

3729939      3732583      3741870      3744980      3745235

3746406

10 mm

**Figure 12**

| CDI | 0.30 | 0.17 | 0.18 | 0.21 | 0.26 | 0.48 |

**Figure 13**

| CDI | 0.70 | 0.42 | 0.31 | 0.31 | 0.19 | 0.19 |

**Figure 14**

| CDI | 0.84 | 0.46 | 0.20 | 0.22 | 0.30 | 0.41 |

**Figure 15**

| CDI | 0.78 | 0.53 | 0.57 | 0.35 | 0.28 | 0.36 |

**Figure 16**

| | Control | 1:1 | 1:2 | 1:4 | 1:6 | 1:8 | 1:10 |
|---|---|---|---|---|---|---|---|
| CDI | | 0.76 | 0.51 | 0.45 | 0.36 | 0.16 | 0.21 |

## Figure 17

| | CDI | 0.78 | 0.62 | 0.26 | 0.22 | 0.25 | 0.24 |

**Figure 18**

| CDI | 2.44 | 0.66 | 0.55 | 0.46 | 0.70 | 0.81 |

**Figure 19**

| CDI | 0.28 | 0.15 | 0.13 | 0.13 | 0.11 | 0.25 |

# Figure 20

| CDI | 0.66 | 0.56 | 0.38 | 0.28 | 0.19 | 0.15 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011047434 A

**Non-patent literature cited in the description**

- **NOVAK et al.** *Anticancer Research,* 2005, vol. 25, 1157-1177

- **ULLMAN-CULLERE ; FOLTZ.** *Lab. Animal Science,* 1999, vol. 49, 319